# FASCICULE DE BREVET EUROPEEN

(11) **EP 1 915 442 B1**
(45) Date de publication et mention de la délivrance du brevet: **01.09.2010**
(21) Numéro de dépôt: 06778826.5
(22) Date de dépôt: 07.07.2006
(51) Int. Cl.: C12N 9/16, C12P 7/18, C12P 3/00, C12N 1/15, C12N 1/19, A23K 1/165

(54) **PHYTASE DE DEBARYOMYCES CASTELLII**
PHYTASE AUS DEBARYOMYCES CASTELLII
DEBARYOMYCES CASTELLII PHYTASE

(30) Priorité: 08.07.2005 FR 0507336
(43) Date de publication de la demande: 30.04.2008
(73) Titulaire: Adisseo France S.A.S., 92160 Antony (FR)
(72) Inventeur: BOZE, Hélène, F-34070 Montpellier (FR); AUMELAS, André, F-34680 Saint Georges d'Orques (FR); MOULIN, Guy, F-34980 Montferrier-sur-Lez (FR)
(74) Mandataire: Jouannic, Nathalie
(86) Numéro de dépôt international: PCT/FR2006/001653
(87) Numéro de publication internationale: WO 2007/006953

(56) Documents cités:
- EP-A- 0 699 762
- LAMBRECHTS C ET AL: "UTILIZATION OF PHYTATE BY SOME YEASTS" BIOTECHNOLOGY LETTERS, KEW, SURREY, GB, vol. 14, no. 1, janvier 1992 (1992-01), pages 61-62, XP002053185 ISSN: 0141-5492 cité dans la demande
- NAKAMURA YOSHIHIRO ET AL: "Secreted phytase activities of yeasts" BIOSCIENCE BIOTECHNOLOGY AND BIOCHEMISTRY, vol. 64, no. 4, avril 2000 (2000-04), pages 841-844, XP002369733 ISSN: 0916-8451
- SEGUEILHA L ET AL: "PURIFICATION AND PROPERTIES OF THE PHYTASE FROM SCHWANNIOMYCES CASTELLII" JOURNAL OF FERMENTATION AND BIOENGINEERING, SOCIETY OF FERMENTATION TECHNOLOGY, JP, vol. 74, no. 1, 1992, pages 7-11, XP002053183 ISSN: 0922-338X cité dans la demande
- VOHRA A ET AL: "PHYTASES: MICROBIAL SOURCES, PRODUCTION, PURIFICATION, AND POTENTIAL BIOTECHNOLOGICAL APPLICATIONS" CRC CRITICAL REVIEWS IN BIOTECHNOLOGY, CRC PRESS, BOCA RATON, FL, US, vol. 23, no. 1, 2003, pages 29-60, XP008060235 ISSN: 0738-8551
- NASI M ET AL: "COMPARISON OF ASPERGILLUS NIGER PHYTASE AND TRICHODERMA REESEI PHYTASE AND ACID PHOSPHATASE ON PHYTATE PHOSPHORUS AVAILABILITY IN PIGS FED ON MAIZE-SOYBEAN MEAL OR BARLEY-SOYBEAN MEAL DIETS" ARCHIV FUER TIERERNAEHRUNG - ARCHIVES OF ANIMAL NUTRITION, AKADEMIE VERLAG, BERLIN, DE, vol. 52, no. 1, 1999, pages 15-27, XP008045959 ISSN: 0003-942X

### SEQUENCE LISTING

<110> ADISSEO FRANCE SAS
<120> Phytase de Debaryomyces castellii
<130> HL/SG/EP-49825
<150> FR05.07336
   <151> 2005-08-07
<160> 18
<170> PatentIn version 3.2
<210> 1
   <211> 2973
   <212> DNA
   <213> Debaryomyces castellii
<220>
   <221> misc_feature
   <222> (782)..(782)
   <223> n is a, c, g, or t
<220>
   <221> CDS
   <222> (1538)..(2923)
<400> 1
   aagattgtgt tagataataa gaagcaaacc cttgatgatt tttttggaaa aaaggatttt   60
   gctcttatag aaagggccgc tgctgttgat aaagaaggtt ctacagaacc tgagattata  120
   aatgatgctt ctaaggaaaa atcagcaagt ccgtcagata gcagaatttc tcgatcagaa  180
   attgaaaaca tgaattctaa cgaagaaaat ctctcgcttt taggtaagaa gcgcttaagc  240
   gcaaaggaaa gaagattgtt aagaaagaat aaaaagaatg actctgctgc tcaaagtgat  300
   gatgatgatg atgtgttaga tccaattaaa caacagttgc aaaaattaaa acttcaagaa  360
   agtaaggcta ctgaaaaaga agctcctaac cagaaaccac ctaatgttag aggtaaaaaa  420
   gccaagctta agaagattgc agctaagtat gcagatcaaa cagaggaaga aagaagattg  480
   agaatggaag cattgggaac tttgaagcaa gttgaacagg aaaagcaaaa tcaattgcaa  540
   aatgatcata agaatgagct caacaatgaa aaggcacaaa tgaatcttga aaagaaaaag  600
   aaacaggaag aaagagaata ccgtaaatat atcatggatg aagtagacga gtcggaatca  660
   tccttgacaa attacttaga aatacttgat tccttcattt caaagccgca gccatctgat  720
   gtgctttctc atccaatcgc ggtgtttgca ccgtggtcct ctctccaaaa attcaaatat  780
   atggtcaaaa tccacccggg ttcaggaaag aagggtaaat gcatcaacga cactcttaac  840
   tacttcacta ctcggaaatt ggatgagctg cgttctgata ccgatttgga ctggctgcat    900
   gaaagagata tgcttaagcg cataaagccc aacgatctta tgggggtgtt taccgttagc    960
   aaagttaaat tagtgctacc aggtggcctg gagtcaaaca aagctctggg tgcgaataag   1020
   aaggcgactg gctccaaaag gaaaaagtga ttaacgcccc aatcggacct ttgttttatg   1080
   tacaattaat tagtttaatt agttcgtatt tgcattgatt tactctttct ctagaatatg   1140
   acctgatagg acactacata ccattcttaa taatagatac atatcccgga ctaatatgtt   1200
   ttgtgtcaaa ttaggaaatc ctctgtcaaa tctgtctaat aacaatgtaa attagtgaga   1260
   ctaattacat tatctacagc acgtgtatca ctactttaag ctttgaccct tgtgacctaa   1320
   aggattcttt tacacgtgtg tggaagcagc agaactattg gacacagacg aaagtgtgct   1380
   tcttcatcga tcaatataga atgcataata agcgaattgt ttttgagtcc caaaaaagca   1440
   gagaactgtt atgaatatgg ttgtgaaaag tataaaagac aaagaagtcc tcgaattgct   1500
   atggatagat tcataagtat caaagtaaat agtcaat atg gtc tca gtc tca aag    1555
                                           Met Val Ser Val Ser Lys
                                           1               5
   tta att aac aat ggt tta ttg ttg gta ggt caa ggt gcc tac caa gat     1603
   Leu Ile Asn Asn Gly Leu Leu Leu Val Gly Gln Gly Ala Tyr Gln Asp
               10                  15                  20
   ttg gct tcc cca caa caa gcg tcc gtc gag caa tat aac atc atc aga     1651
   Leu Ala Ser Pro Gln Gln Ala Ser Val Glu Gln Tyr Asn Ile Ile Arg
           25                  30                  35
   ttc tta gga gga gct gct cca tat atc caa aac aaa ggg ttc ggt atc     1699
   Phe Leu Gly Gly Ala Ala Pro Tyr Ile Gln Asn Lys Gly Phe Gly Ile
       40                  45                  50
   tct act gat atc cca gat caa tgt act ctt gag caa gtc caa ttg ttc     1747
   Ser Thr Asp Ile Pro Asp Gln Cys Thr Leu Glu Gln Val Gln Leu Phe
   55                  60                  65                  70
   agc aga cat ggt gaa aga tac cca tcc act ggc tcc gga aag aaa tat     1795
   Ser Arg His Gly Glu Arg Tyr Pro Ser Thr Gly Ser Gly Lys Lys Tyr
                   75                  80                  85
   aag gct gta tat gaa aag ttg atg tca tac aac ggt act ttc aag gga     1843
   Lys Ala Val Tyr Glu Lys Leu Met Ser Tyr Asn Gly Thr Phe Lys Gly
               90                  95                  100
   gaa ttg gct ttc ctc aat gac gat tat gaa tat ttt gtt cct gac tca     1891
   Glu Leu Ala Phe Leu Asn Asp Asp Tyr Glu Tyr Phe Val Pro Asp Ser
           105                 110                 115
   gtg tat ctt gaa aag gaa aca tca cca aag aat tct gac agt atc tac     1939
   Val Tyr Leu Glu Lys Glu Thr Ser Pro Lys Asn Ser Asp Ser Ile Tyr
       120                 125                 130
   gct ggt acc act gat gcc atg aag cat ggt atc gcc ttt aga acc aag     1987
   Ala Gly Thr Thr Asp Ala Met Lys His Gly Ile Ala Phe Arg Thr Lys
   135                 140                 145                 150
   tac ggt gaa ttg ttc gat act aat gac act ctt cca gtt ttc act tcc      2035
   Tyr Gly Glu Leu Phe Asp Thr Asn Asp Thr Leu Pro Val Phe Thr Ser
                   155                 160                 165
   aat tct ggt aga gta tat caa acc tct caa tac ttt gca aga ggt ttt      2083
   Asn Ser Gly Arg Val Tyr Gln Thr Ser Gln Tyr Phe Ala Arg Gly Phe
               170                 175                 180
   atg gga gat gat ttc agt aac gat acc gtc aaa acc aat att atc tct      2131
   Met Gly Asp Asp Phe Ser Asn Asp Thr Val Lys Thr Asn Ile Ile Ser
           185                 190                 195
   gaa gac gct gat atg ggt gcc aac tca tta aca cca aga gat ggt tgt      2179
   Glu Asp Ala Asp Met Gly Ala Asn Ser Leu Thr Pro Arg Asp Gly Cys
       200                 205                 210
   ttc aac tac aat gaa aat gct aac act gct att gtt gat gaa tac act      2227
   Phe Asn Tyr Asn Glu Asn Ala Asn Thr Ala Ile Val Asp Glu Tyr Thr
   215                 220                 225                 230
   act gaa tat tta act aaa gct ctt aac aga ttt aaa gct tct aat cct      2275
   Thr Glu Tyr Leu Thr Lys Ala Leu Asn Arg Phe Lys Ala Ser Asn Pro
                   235                 240                 245
   ggt ttg aac att act gaa gat gat gtt tct aat ctt ttt gga tac tgt      2323
   Gly Leu Asn Ile Thr Glu Asp Asp Val Ser Asn Leu Phe Gly Tyr Cys
               250                 255                 260
   gct tat gaa tta aat gtt aaa gga gct tct cca atg tgt gat atc ttt      2371
   Ala Tyr Glu Leu Asn Val Lys Gly Ala Ser Pro Met Cys Asp Ile Phe
           265                 270                 275
   act aat gaa gag ttc att caa tac tct tac agt gtt gat ctt gat gac      2419
   Thr Asn Glu Glu Phe Ile Gln Tyr Ser Tyr Ser Val Asp Leu Asp Asp
       280                 285                 290
   tat tac tcc aac agt gca ggt aat aat atg act aga gtc atc ggt tca      2467
   Tyr Tyr Ser Asn Ser Ala Gly Asn Asn Met Thr Arg Val Ile Gly Ser
   295                 300                 305                 310
   aca tta tta aac gca tcc ttg gaa tta tta aac cat gac aaa aat gag      2515
   Thr Leu Leu Asn Ala Ser Leu Glu Leu Leu Asn His Asp Lys Asn Glu
                   315                 320                 325
   aat aag att tgg tta tct ttc act cac gat act gat att gaa att ttt      2563
   Asn Lys Ile Trp Leu Ser Phe Thr His Asp Thr Asp Ile Glu Ile Phe
               330                 335                 340
   cat tct gct att ggt att ctt atc cct gat gaa gat tta cca gtt gat      2611
   His Ser Ala Ile Gly Ile Leu Ile Pro Asp Glu Asp Leu Pro Val Asp
           345                 350                 355
   tac act cca ttc cca tct cct tat tct cac gtt gga att act cct caa      2659
   Tyr Thr Pro Phe Pro Ser Pro Tyr Ser His Val Gly Ile Thr Pro Gln
       360                 365                 370
   ggt gct aga act att att gaa aag tac gcg tgt ggt aat gaa tct tat      2707
   Gly Ala Arg Thr Ile Ile Glu Lys Tyr Ala Cys Gly Asn Glu Ser Tyr
   375                 380                 385                 390
   gtt aga tat gtt atc aat gat gct gtt att cca att aag aag tgt agc      2755
   Val Arg Tyr Val Ile Asn Asp Ala Val Ile Pro Ile Lys Lys Cys Ser
                   395                 400                 405
   tct ggt cct ggt ttc tca tgt aat ctt aat gat tat aat gat tat gtt      2803
   Ser Gly Pro Gly Phe Ser Cys Asn Leu Asn Asp Tyr Asn Asp Tyr Val
               410                 415                 420
   gct gaa aga gtt gca ggt acc aac tat gtt gaa caa tgt ggt aat aac      2851
   Ala Glu Arg Val Ala Gly Thr Asn Tyr Val Glu Gln Cys Gly Asn Asn
           425                 430                 435
   aat gct tca gct gtt aca ttc tac tgg gat tac gaa act act aac tac      2899
   Asn Ala Ser Ala Val Thr Phe Tyr Trp Asp Tyr Glu Thr Thr Asn Tyr
       440                 445                 450
   acc gct tcc ctt atc aac agt taa aatttttctt tcttttactt tcttcttcaa     2953
   Thr Ala Ser Leu Ile Asn Ser
   455                 460
   aacaagtttc tatttctttt                                                 2973
<210> 2
   <211> 461
   <212> PRT
   <213> Debaryomyces castellii
<400> 2
   Met Val Ser Val Ser Lys Leu Ile Asn Asn Gly Leu Leu Leu Val Gly
   1               5                   10                  15
   Gln Gly Ala Tyr Gln Asp Leu Ala Ser Pro Gln Gln Ala Ser Val Glu
               20                  25                  30
   Gln Tyr Asn Ile Ile Arg Phe Leu Gly Gly Ala Ala Pro Tyr Ile Gln
           35                  40                  45
   Asn Lys Gly Phe Gly Ile Ser Thr Asp Ile Pro Asp Gln Cys Thr Leu
       50                  55                  60
   Glu Gln Val Gln Leu Phe Ser Arg His Gly Glu Arg Tyr Pro Ser Thr
   65                  70                  75                  80
   Gly Ser Gly Lys Lys Tyr Lys Ala Val Tyr Glu Lys Leu Met Ser Tyr
                   85                  90                  95
   Asn Gly Thr Phe Lys Gly Glu Leu Ala Phe Leu Asn Asp Asp Tyr Glu
           100                 105                 110
   Tyr Phe Val Pro Asp Ser Val Tyr Leu Glu Lys Glu Thr Ser Pro Lys
           115                 120                 125
   Asn Ser Asp Ser Ile Tyr Ala Gly Thr Thr Asp Ala Met Lys His Gly
       130                 135                 140
   Ile Ala Phe Arg Thr Lys Tyr Gly Glu Leu Phe Asp Thr Asn Asp Thr
   145                 150                 155                 160
   Leu Pro Val Phe Thr Ser Asn Ser Gly Arg Val Tyr Gln Thr Ser Gln
                   165                 170                 175
   Tyr Phe Ala Arg Gly Phe Met Gly Asp Asp Phe Ser Asn Asp Thr Val
               180                 185                 190
   Lys Thr Asn Ile Ile Ser Glu Asp Ala Asp Met Gly Ala Asn Ser Leu
           195                 200                 205
   Thr Pro Arg Asp Gly Cys Phe Asn Tyr Asn Glu Asn Ala Asn Thr Ala
       210                 215                 220
   Ile Val Asp Glu Tyr Thr Thr Glu Tyr Leu Thr Lys Ala Leu Asn Arg
   225                 230                 235                 240
   Phe Lys Ala Ser Asn Pro Gly Leu Asn Ile Thr Glu Asp Asp Val Ser
                   245                 250                 255
   Asn Leu Phe Gly Tyr Cys Ala Tyr Glu Leu Asn Val Lys Gly Ala Ser
               260                 265                 270
   Pro Met Cys Asp Ile Phe Thr Asn Glu Glu Phe Ile Gln Tyr Ser Tyr
           275                 280                 285
   Ser Val Asp Leu Asp Asp Tyr Tyr Ser Asn Ser Ala Gly Asn Asn Met
       290                 295                 300
   Thr Arg Val Ile Gly Ser Thr Leu Leu Asn Ala Ser Leu Glu Leu Leu
   305                 310                 315                 320
   Asn His Asp Lys Asn Glu Asn Lys Ile Trp Leu Ser Phe Thr His Asp
                   325                 330                 335
   Thr Asp Ile Glu Ile Phe His Ser Ala Ile Gly Ile Leu Ile Pro Asp
               340                 345                 350
   Glu Asp Leu Pro Val Asp Tyr Thr Pro Phe Pro Ser Pro Tyr Ser His
           355                 360                 365
   Val Gly Ile Thr Pro Gln Gly Ala Arg Thr Ile Ile Glu Lys Tyr Ala
       370                 375                 380
   Cys Gly Asn Glu Ser Tyr Val Arg Tyr Val Ile Asn Asp Ala Val Ile
   385                 390                 395                 400
   Pro Ile Lys Lys Cys Ser Ser Gly Pro Gly Phe Ser Cys Asn Leu Asn
                   405                 410                 415
   Asp Tyr Asn Asp Tyr Val Ala Glu Arg Val Ala Gly Thr Asn Tyr Val
               420                 425                 430
   Glu Gln Cys Gly Asn Asn Asn Ala Ser Ala Val Thr Phe Tyr Trp Asp
           435                 440                 445
   Tyr Glu Thr Thr Asn Tyr Thr Ala Ser Leu Ile Asn Ser
       450                 455                 460
<210> 3
   <211> 7
   <212> PRT
   <213> Séquence artificielle
<220>
   <221> misc_feature
   <222> (4)..(4)
   <223> Xaa can be any naturally occurring amino acid
<220>
   <221> misc_feature
   <222> (6)..(6)
   <223> Xaa can be any naturally occurring amino acid
<400> 3
   Arg His Gly Xaa Arg Xaa Pro
   1               5
<210> 4
   <211> 7
   <212> PRT
   <213> Debaryomyces castellii
<400> 4
   Arg His Gly Glu Arg Tyr Pro
   1               5
<210> 5
   <211> 19
   <212> DNA
   <213> Séquence artificielle
<220>
   <221> misc_feature
   <223> Amorce Phyt-Nter-for
<400> 5
   tcaarttrat haayaaygg 19
<210> 6
   <211> 20
   <212> DNA
   <213> Séquence artificielle
<220>
   <221> misc_feature
   <223> Amorce Phyt-pepl-rev
<220>
   <221> misc_feature
   <222> (3)..(3)
   <223> n is a, c, g, or t
<400> 6
   ggnacraart aytcrtartc 20
<210> 7
   <211> 22
   <212> DNA
   <213> Séquence artificielle
<220>
   <221> misc_feature
   <223> Amorce AP1
<400> 7
   gtaatacgac tcactatagg gc 22
<210> 8
   <211> 19
   <212> DNA
   <213> Séquence articielle
<220>
   <221> misc_feature
   <223> Amorce AP2
<400> 8
   actatagggc acgcgtggt 19
<210> 9
   <211> 27
   <212> DNA
   <213> Séquence articielle
<220>
   <221> misc_feature
   <223> Amorce 5phyt-spe-1
<400> 9
   tatggagcag ctcctcctaa gaatctg 27
<210> 10
   <211> 29
   <212> DNA
   <213> Séquence articielle
<220>
   <221> misc_feature
   <223> Amorce 5phyt-spe-2
<400> 10
   atgatgttat attgctcgac ggacgcttg 29
<210> 11
   <211> 27
   <212> DNA
   <213> Séquence articielle
<220>
   <221> misc_feature
   <223> Amorce 3phyt-spe-1
<400> 11
   tatggagcag ctcctcctaa gaatctg 27
<210> 12
   <211> 29
   <212> DNA
   <213> Séquence articielle
<220>
   <221> misc_feature
   <223> Amorce 3phyt-spe-2
<400> 12
   ctggctccgg aaagaaatat aaggctgta 29
<210> 13
   <211> 30
   <212> DNA
   <213> Séquence articielle
<220>
   <221> misc feature
   <223> Amorce 3bisphyt-spe-1
<400> 13
   gaagtgtagc tctggtcctg gtttctcatg 30
<210> 14
   <211> 30
   <212> DNA
   <213> Séquence articielle
<220>
   <221> misc_feature
   <223> Amorce 3bisphyt-spe-2
<400> 14
   atgttgctga aagagttgca ggtaccaact 30
<210> 15
   <211> 32
   <212> DNA
   <213> Séquence articielle
<220>
   <221> misc_feature
   <223> Amorce phytDC-PstI-for
<400> 15
   gcactgcagt ctcagtctca aagttaatta ac 32
<210> 16
   <211> 33
   <212> DNA
   <213> Séquence articielle
<220>
   <221> misc_feature
   <223> Amorce phytDC-XbaI-rev
<400> 16
   agttctagat taactgttga taagggaagc ggt 33
<210> 17
   <211> 7
   <212> PRT
   <213> Debaryomyces castellii
<400> 17
   Ser Lys Leu Ile Asn Asn Gly
   1               5
<210> 18
   <211> 7
   <212> PRT
   <213> Debaryomyces castellii
<400> 18
   Pro Val Phe Tyr Glu Tyr Asp
   1               5

## Description

L'invention concerne une phytase de *Debaryomyces castellii,* le gène codant cette phytase, des organismes hôtes recombinants exprimant cette phytase ainsi que ses applications dans le domaine de la nutrition animale notamment.

Les sels de l'acide phytique (myo-inositol hexakis phosphate) ou phytates (myo-inositol hexakis(dihydrogène phosphate) sont la forme majeure de stockage du phosphore dans les céréales, les plantes légumineuses et oléagineuses. Ils constituent ainsi la source principale de phosphore dans les aliments pour animaux à base de plantes, composants principaux des régimes des animaux monogastriques (volailles et porcs). Cependant, la biodisponibilité de ce phosphore dans les aliments est limitée pour ces animaux. En effet, ils ne possèdent pas les enzymes intestinales dégradant les phytates en quantité suffisante pour permettre l'hydrolyse des phytates et fournir ainsi les quantités de phosphate inorganique qui leur sont nécessaires. Dans le contexte de la nutrition animale, deux caractéristiques de l'acide phytique jouent ainsi un rôle important : (1) les animaux monogastriques sont faiblement capables de dégrader l'acide phytique dans l'appareil digestif; (2) l'acide phytique est un facteur antinutritionnel, qui forme des complexes avec les protéines et les ions (Fe³⁺, Ca²⁺, Zn²⁺, Mg²⁺) et diminue donc la disponibilité de ces éléments.

Les rations alimentaires des volailles et des porcs doivent donc être supplémentées en phosphate inorganique alors que le phosphore des phytates est excrété et contribue à l'eutrophisation des eaux de surface dans les zones d'élevage intensif d'animaux monogastriques.

La complémentation des rations des animaux monogastriques par des enzymes est une solution actuellement employée pour améliorer la biodisponibilité du phosphore liée au phytate et pour diminuer la supplémentation des aliments avec du phosphore inorganique et réduire ainsi l'excrétion de phosphore dans les zones d'élevage intensif.

Les phytases (myo-inositol hexakis phosphate 3- et 6-phosphohydrolases EC 3.1.3.8 et 3.1.3.26) font partie de la famille des histidines phosphatases acides. Elles catalysent l'hydrolyse du *myo*-inositol hexakisphosphate (acide phytique, InsP₆) en monophosphate inorganique et en *myo*-inositol phosphate de degré de phosphorylation inférieur (InsP₅ à InsP₁) et en *myo*-inositol libre dans certains cas.

Ces enzymes utilisées comme additif en alimentation animale, permettent d'une part d'augmenter la disponibilité du phosphore phytique, d'autre part d'améliorer la digestibilité des aliments. De plus, la libération de phosphate phytique diminue considérablement les coûts dus à la supplémentation en phosphate, ainsi que la pollution provoquée par un excès de phosphates excrétés.

Les phytases sont produites par une grande variété d'organismes : plantes, animaux, et surtout microorganismes. Parmi les microorganismes producteurs de phytases on citera notamment : les champignons des genres *Aspergillus, Penicillium, Mucor* et *Rhizopus,* les *bactéries : Pseudomonas* sp., *Klebsiella* sp., *Escherichia coli, Enterobacter* sp., *Bacillus subtilis* et levures : *Saccharomyces cerevisiae, Candida tropicalis, Torulopsis candida, Debaryomyces castellii, Debaryomyces occidentalis, Kluyveromyces fragilis* et *Schwanniomyces castellii.*

En ce qui concerne les levures, Lambrechts et al. (Biotechnology Letters, Vol. 14, no. 1, 61-66, 1992) ont identifié différentes souches ayant une activité phytase mais n'ont pas caractérisé les phytases correspondantes. L'activité la plus élevée a été mesurée pour la souche CBS2863 de *Schwanniomyces castellii.* Des activités phytase ont également été observées pour *Candida brumptii* CBS 6145, C. *tropicalis* CBS5696, *Debaryomyces castellii* CBS 2923, *Kluyveromyces fragilis* n°111, *K. fragilis* CBS 1555, K. fragilis CBS 5795, *Saccharomyces cerevisiae* CBS 1253, *Schwanniomyces castellii* CBS 2863, *Torulopsis candida* CBS 940, C. *melibiosica* CBS 584, *K. lactis* CBS 2359, *T. bovina* CBS 2760 et *Zygosaccharomyces fennentati* CBS6772.

De même, Nakamura Yoshihiro et al. (Biosci. Biotechnol. Biochem., Vol. 64, no. 4, 841-844, 2000) ont identifié différentes phytases, mais ne les ont pas caractérisées.

Il est à noter en outre que la classification des levures a été revue. Selon la nouvelle classification (Nakase T., Suzuki M., Phaff H.J. and Kurtzman C.P., 1998 p157-167 in C. P. Kurtzman and J.W. Fell (ed), The Yeasts, A taxonomic study, 4eme Ed. Elsevier Sci. Publication Amsterdam), la souche CBS 2923 correspond à *Debaryomyces castellii Capriotti* et la souche CBS 2863 correspond à *Debaryomyces occidentalis Klocker var. occidentalis,* synonymes *Schwanniomyces castellii Capriotti* et *Schwanniomyces occidentalis Kloker.* Les inventeurs ont montré que l'alignement du polypeptide de la SEQ ID No. 2, c'est-à-dire la séquence de la phytase de *Debaryomyces castellii* CBS 2923, conduit à un pourcentage d'identité de 69,2% avec la séquence de la phytase de *Schwanniomyces occidentalis* CBS 2863.

De nombreuses phytases de microorganismes ont déjà été étudiées et utilisées dans diverses applications agro-industrielles. Cependant, l'utilisation croissante de phytases comme additif dans de nombreuses applications biotechnologiques, telle que l'alimentation animale augmente l'intérêt : (1) d'isoler de nouveaux microorganismes producteurs efficaces de phytases, (2) d'obtenir des phytases performantes, c'est à dire présentant une efficacité à libérer des phosphates de l'aliment dans le tractus digestif, une stabilité à la chaleur pendant le processus de fabrication de l'aliment et pendant le stockage, et un faible coût de production.

En outre, la plupart des phytases décrites à ce jour n'hydrolysent que partiellement l'acide phytique et certaines avec des cinétiques très lentes. De plus, leur activité d'hydrolyse dépend fortement de leurs conditions de mise en oeuvre.

A ce jour, parmi les levures, seule la phytase de la levure *Schwanniomyces occidentalis* a été décrite comme hydrolysant tous les groupements phosphate du phytate (EP 0 699 762 et Segueilha L., Lambrechts C., Boze H., Moulin G., Galzy P., (1992) Purification and properties of a phytase from Schwanniomyces castelliii, J. Ferm. Bioeng., 74, 7-11). Cependant, la phytase de *Schwanniomyces occidentalis* est sensible aux cations (ZnCl₂ et CuCl₂ notamment) ce qui n'est pas favorable à une utilisation en alimentation animale car ces cations sont présents dans le bol alimentaire (Segueilha *et al*., 1992). De plus, cette enzyme est active dans une gamme de pH de 2.7 à 5 (EP 0 699 762) et le pCMB inhibe fortement l'activité de la phytase de *Schwanniomyces occidentalis,* ce qui suggère que des groupements SH (ponts disulfures) sont impliqués dans le site actif de l'enzyme (Segueilha *et al.,* 1992). En outre, la biosynthèse de cette phytase nécessite la présence de phytates et de sels de calcium (EP 0 699 762). L'activité de cette enzyme dépend donc fortement de l'environnement dans lequel elle se trouve. Le problème que se propose de résoudre la présente invention consiste à proposer une phytase hydrolysant tous les groupements phospate du phytate et dont l'activité dépend peu des conditions de mise en oeuvre de l'enzyme.

Ce problème est résolu par la phytase de la SEQ ID No. 2. De façon avantageuse, cette phytase est capable d'hydrolyser la totalité des groupements phosphate du phytate jusqu'à la libération du myo-inositol. Elle possède également un large spectre d'activité et hydrolyse ainsi de nombreux substrats. De façon avantageuse, la biosynthèse de cette phytase est induite par les phytates, même en absence de sels de calcium. Avantageusement, cette phytase est active dans une plage de pH élargie (pH 3 à 6,5) et elle n'est pas sensible aux cations ou au pCMB. L'enzyme est également thermostable jusqu'à 66°C. L'enzyme de la présente invention est donc extrèmement robuste ce qui est favorable à une utilisation en alimentation animale mais aussi dans d'autres applications industrielles.

L'invention se rapporte aussi à des phytases similaires ou homologues, à des variants et à des fragments de la phytase de la SEQ ID No. 2 conservant les mêmes propriétés.

### Description des séquences

SEQ ID No. 1 : Séquence génomique du gène de la phytase de *Debaryomyces castellii* CBS 2923.
SEQ ID No. 2 : Séquence de la phytase de *Debaryomyces castellii* CBS 2923.
SEQ ID No. 3 : Séquence consensus des phosphatases acides.
SEQ ID No. 4 : Motif de la phytase de *Debaryomyces castellii* correspondant à la séquence consensus des phosphatases acides de la SEQ ID No. 3.
SEQ ID Nos. 5-16 : Amorces pour clonage
SEQ ID Nos. 17-18 : Peptides de la phytase de *Debaryomyces castellii* utilisés pour le clonage du gène

### Description de l'invention

L'invention a pour objet des polypeptides comprennant un polypeptide choisi parmi les polypeptides suivants:
- le polypeptide de la SEQ ID No.
- un fragment du polypeptide de la SEQ ID No. 2 ayant une activité phytase,
- un polypeptide ayant une activité phytase et présentant au moins 90 % d'identité avec le polypeptide de la SEQ ID No. 2.

L'invention a aussi pour objet des polynucléotides codant pour une activité phytase choisis parmi les polynucléotides suivants :
- le polynucléotide dont la séquence est comprise entre la position 1538 et la position 2923 de la SEQ ID No. 1,
- un polynucléotide codant pour un polypeptide selon la revendication 1.

L'invention se rapporte aussi aux polynucléotides ayant la séquence de la SEQ ID No. 1 ou la séquence complémentaire à la SEQ ID No. 1.

L'invention concerne aussi des cassettes d'expression comprenant dans le sens de la transcription:
- un promoteur fonctionnel dans un organisme hôte;
- un polynucléotide selon l'invention; et
- une séquence terminatrice dans le même organisme hôte.

L'invention concerne également des vecteurs comprenant un polynucléotide selon l'invention et/ou une cassette d'expression selon l'invention.

Un autre objet de la présente invention est un organisme hôte transformé avec un polynucléotide selon l'invention, une cassette d'expression selon l'invention et/ou un vecteur selon l'invention.

L'organisme hôte est *Pichia pastoris.*

L'invention a pour objet des additifs nutritionnels pour animaux comprenant un polypeptide selon l'invention.

L'invention a aussi pour objet des additifs nutritionnels pour animaux comprenant un organisme hôte selon l'invention et/ou un moût de fermentation d'un organisme hôte selon l'invention.

Dans un mode de réalisation de l'invention, l'additif nutritionnel pour animaux se présente sous forme liquide ou sous forme de poudre.

L'invention concerne les aliments pour animaux comprenant une base nutritionnelle pour animaux et un additif nutritionnel pour animaux selon l'invention.

L'invention concerne également des aliments pour animaux comprenant un polypeptide selon l'invention, un organisme hôte selon l'invention et/ou un moût de fermentation d'un organisme hôte selon l'invention.

L'invention se rapporte également à l'utilisation d'un polypeptide selon l'invention ou d'un organisme hôte selon l'invention pour la fabrication d'un additif nutritionnel pour animaux ou d'un aliment pour animaux.

Un autre objet de la présente invention est l'utilisation d'un polypeptide selon l'invention ou d'un organisme hôte selon l'invention pour l'hydrolyse du myo-inositol hexakisphosphate en monophosphate inorganique, en myo-inositol de degré de phosphorylation inférieur et en myo-inositol libre.

### Polypeptides

La présente invention concerne donc des polypeptides ayant une activité phytase. De préférence, ces polypetides sont isolés de *Debaryomyces castellii.*

La phytase de *Debaryomyces castellii* CBS 2923 est représentée à la SEQ ID No.2.

On entend par "phytase", les myo-inositol hexakis phosphate 3- et 6-phosphohydrolases (EC 3.1.3.8 et 3.1.3.26). Ces enzymes catalysent l'hydrolyse du myo-inositolhexakiphosphate (acide phytique, InsP₆) en monophosphate inorganique et en myo-inositolphosphate de degré inférieur (InsP₅ à InsP₁) et en myo-inositol libre pour certaines phytases.

La phytase de *Debaryomyces castellii* de la SEQ ID No. 2 est une 3-phytase. En outre, elle possède la capacité remarquable d'hydrolyser la totalité des liaisons phosphate de l'acide phytique.

La phytase de la SEQ ID No. 2 comprend le motif RHGERYP (SEQ ID No. 4) correspondant à la séquence consensus RHGXRXP (SEQ ID No. 3) présente dans le site actif de nombreuses phosphatases acides. Ce motif est retrouvé aux acides aminés 72-78 de la SEQ ID No. 2. La phytase de la SEQ ID No. 2 possède également le motif HD présent chez de nombreuses phytases. Ce motif est retrouvé dans la partie C-terminale aux acides aminés 335-336 de la SEQ ID No. 2.

Dans un mode de réalisation préfére de l'invention, les polypeptides selon l'invention possèdent le motif RHGERYP ou un autre motif correspondant à la séquence consensus RHGXRXP. Préférentiellement, les polypeptides selon l'invention possèdent également un motif HD.

Dans un mode de réalisation préféré, les polypeptides selon l'invention sont glycosylés. Le polypeptide de la SEQ ID No. 2 possède notamment des sites putatifs de N-glycosylation aux acides aminés Asn 97, Asn 158, Asn 189, Asn 249, Asn 303, Asn 314, Asn 387, Asn 439 et Asn 453 ; et des sites putatifs de O-glycosylation aux acides aminés Thr 165, Ser 168, Thr 360 et Ser 364. Dans un mode de réalisation préféré, le polypeptide de la SEQ ID No. 2 est glycosylé à un ou plusieurs de ces sites putatifs de N-glycosylation et de O-glycosylation. Dans un mode de réalisation, les polypeptides selon l'invention sont glycosylés.

La phytase de la SEQ ID No. 2 possède également 8 cystéines susceptibles de former 4 ponts disulfures : Cys 62, Cys 214, Cys 262, Cys 275, Cys 385, Cys 405, Cys 413 et Cys 435. Dans un mode de réalisation, les polypeptides selon l'invention portent au moins un pont disulfure.

La phytase de *Debaryomyces castellii* est une enzyme secrétée par cette levure dans son environnement extracellulaire.

Pour l'expression et la secretion par un organisme hôte recombinant, la phytase de la SEQ ID No. 2 peut être fusionnée à son extrémité N-terminale avec un peptide signal reconnu par cet organisme hôte.

L'invention se rapporte également à des fragments du polypeptide de la SEQ ID No. 2 conservant une activité phytase.

Le terme "fragment" d'un polypeptide désigne un polypeptide comprenant une partie mais pas la totalité du polypeptide dont il est dérivé. L'invention concerne ainsi un polypeptide comprenant un fragment d'au moins 100, 200, 300 ou 400 acides aminés du polypeptide de la SEQ ID No. 2.

Ce fragment du polypeptide de la SEQ ID No. 2 conserve son activité phytase. L'invention concerne donc les fragments biologiquement actifs du polypeptide de la SEQ ID No. 2.

Le terme "fragment biologiquement actif' désigne un fragment d'un polypeptide conservant la fonction du polypeptide dont il est dérivé. Les fragments biologiquement actifs du polypeptide de la SEQ ID No. 2 conservent ainsi les propriétés catalytiques de la phytase de *Debaryomyces castellii* de la SEQ ID No. 2. Les méthodes de préparation de fragments d'un polypeptide ainsi que les techniques de mesure de l'activité phytase sont bien connues de l'homme du métier.

L'invention a pour objet des polypeptides ayant une activité phytase et présentant au moins 90 % d'identité avec le polypeptide de la SEQ ID No. 2. L'invention a aussi pour objet des polypeptides présentant au moins 80 %, 90%, 95%, 98% et préférentiellement au moins 99% d'acides aminés identiques avec le polypeptide de la SEQ ID No. 2.

De préférence, ces polypeptides ont les mêmes propriétés et notamment les mêmes propriétés catalytiques que les polypeptides de la SEQ ID No. 2. Préférentiellement, ces polypeptides sont isolés d'autres souches de *Debaryomyces castellii* ou d'autres levures. Alternativement, ces polypeptides peuvent être obtenus par des techniques de mutagenèse dirigée par exemple.

Par acides aminés identiques, on entend des acides aminés invariants entre deux séquences. Ces polypeptides peuvent présenter une délétion, une addition ou une substitution d'au moins un acide aminé par rapport au polypeptide de la SEQ ID No. 2.

L'invention a également pour objet des polypeptides présentant au moins, 90%, 95%, 98% et préférentiellement au moins 99% de similarité avec le polypeptide de la SEQ ID No. 2. De préférence, ces polypeptides ont les mêmes propriétés et notamment les mêmes propriétés catalytiques que les polypeptides de la SEQ ID No. 2. Préférentiellement, ces polypeptides sont isolés d'autres souches de *Debaryomyces castellii* ou d'autres levures. Alternativement, ces polypeptides peuvent être obtenus par des techniques de mutagenèse dirigée par exemple.

Par similarité, on entend la mesure de la ressemblance entre séquences protéiques ou nucléiques. Ces polypeptides peuvent présenter une délétion, une addition ou une substitution d'au moins un acide aminé par rapport au polypeptide de la SEQ ID No. 2. Le degré de similarité entre deux séquences, quantifié par un score, est basé sur le pourcentage d'identités et/ou de substitution conservatrices des séquences.

Les méthodes de mesure et d'identification du degré d'identité et du degré de similarité entre polypeptides sont connues de l'homme du métier. On peut employer par exemple Vector NTi 9.1.0, programme d'alignement AlignX (Clustal W algorithm) (Invitrogen INFORMAX, http://www.invitrogen.com). De préférence, on utilise les paramètres par défaut.

Les polypeptides selon l'invention sont isolés ou purifiés de leur environnement naturel. Les polypeptides peuvent être préparés au moyen de différents procédés. Ces procédés sont notamment la purification à partir de sources naturelles telles que des cellules exprimant naturellement ces polypeptides, la production de polypeptides recombinants par des cellules hôtes appropriées et leur purification ultérieure, la production par synthèse chimique ou, enfin, une combinaison de ces différentes approches. Ces différents procédés de production sont bien connus de l'homme du métier. Ainsi, les phytases de la présente invention peuvent être isolées à partir de *Debaryomyces castellii.* Dans un autre mode de réalisation, les phytases de la présente invention sont isolées à partir d'organismes hôtes recombinants exprimant une phytase selon l'invention.

L'invention a également pour objet des protéines de fusion, des protéines recombinantes ou des protéines chimères comprenant les polypeptides selon l'invention. Le terme "polypeptide" désigne également des protéines ainsi que des polypeptides modifiés.

Les polypeptides selon la présente invention ont une activité phytase. Préférentiellement, les polypeptides présentent une activité 3-phytase et possèdent la capacité d'hydrolyser la totalité des liaisons phosphate de l'acide phytique.

### Polynucléotides

L'invention concerne aussi des polynucléotides codant pour une phytase selon l'invention. De préférence, ces polynucléotides codent la phytase de la SEQ ID No. 2 de *Debaryomyces castellii.*

Selon la présente invention, on entend par "polynucléotide " une chaine nucléotidique simple brin ou son complémentaire ou une chaine nucléotidique double brin pouvant être de type ADN ou ARN. De préférence, les polynucléotides de l'invention sont de type ADN, notamment d'ADN double brin. Le terme "polynucléotide" désigne également les polynucléotides modifiés.

Les polynucléotides de la présente invention sont isolés ou purifiés de leur environnement naturel. De préférence, les polynucléotides de la présente invention peuvent être préparés par les techniques classiques de biologie moléculaire telles que décrites par Sambrook et al. (Molecular Cloning : A Laboratory Manual, 1989) ou par synthèse chimique.

Dans un premier mode de réalisation, l'invention se rapporte au polynucléotide dont la séquence est comprise entre la position 1538 et la position 2923 de la SEQ ID No. 1. Ce polynucléotide code la phytase de la SEQ ID No. 2 de *Debaryomyces castellii.*

Par nucléotides identiques, on entend des nucléotides invariants entre deux séquences. Ces polynucléotides peuvent présenter une délétion, une addition ou une substitution d'au moins un nucléotide par rapport au polynucléotide de référence.

Par similarité, on entend la mesure de la ressemblance entre séquences protéiques ou nucléiques. Ces polynucléotides peuvent présenter une délétion, une addition ou une substitution d'au moins un nucléotide par rapport au polynucléotide de référence. Le degré de similarité entre deux séquences, quantifié par un score, est basé sur le pourcentage d'identités et/ou de substitution conservatrices des séquences.

Les méthodes de mesure et d'identification du degré d'identité et du degré de similarité entre les séquences d'acides nucléiques sont bien connues de l'homme du métier. On peut employer par exemple Vector NTi Vector NTi 9.1.0, programme d'alignement AlignX (Clustal W algorithm) (Invitrogen INFORMAX, http://www.invitrogen.com). De préférence, on utilise les paramètres par défaut.

Préférentiellement, les polynucléotides présentant un degré d'identité ou un degré de similarité avec un polynucléotide de référence conservent la fonction de la séquence de référence. Dans le cas présent, les polynucléotides codent pour une activité phytase.

Par " séquence capable de s'hybrider de manière sélective ", on entend selon l'invention les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont bien connues de l'homme du métier. En général la température d'hybridation et de lavage est inférieure d'au moins 5°C au Tm de la séquence de référence à un pH donné et pour une force ionique donnée. Typiquement la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant: 6X SSC, 50 mM Tris-HCl (pH 7.5), 1 mM EDTA, 0.02% PVP, 0.02% Ficoll, 0.02% BSA, 500 µg/ml sperme de saumon denaturé DNA. Les lavages sont par exemple réalisés successivement à faible stringence dans un tampon 2X SSC, 0,1%SDS, à moyenne stringence dans un tampon 0,5X SSC, 01%SDS et à forte stringence dans un tampon 0,1X SSC, 0,1%SDS. L'hybridation peut bien entendu être effectuée selon d'autres méthodes usuelles bien connues de l'homme du métier (voir notamment Sambrook et al., Molecular Cloning : A Labratory Manual, 1989).

L'invention se rapporte de manière générale aux polynucléotides codant pour les polypeptides selon l'invention. En raison de la dégénerescence du code génétique, différents polynucléotides peuvent coder pour un même polypeptide.

Un autre objet de la présente invention est un polynucléotide dont la séquence est représentée à la SEQ ID No. 1. Le polynucléotide de la SEQ ID No. 1 comprend des séquences flanquant le cadre ouvert de lecture (ORF) du gène de la phytase de *Debaryomyces castellii.* Il s'agit notamment des séquences promotrices et terminatrices de ce gène. Ce gène peut être exprimé à partir de ses séquences régulatrices homologues notamment pour une surexpression dans *Debaryomyces castellii* ou dans d'autres levures.

Dans un autre mode de réalisation, ce gène peut être exprimé dans différents organismes hôtes tels que les bactéries, les levures et les champignons par exemple. Le gène codant la phytase de la SEQ ID No. 2 peut être exprimé dans un organisme hôte sous le contrôle du promoteur de la SEQ ID No. 1 de la présente invention ou sous le contrôle d'un promoteur hétérologue.

### Cassettes d'expression

Selon un mode de réalisation de l'invention, un polynucléotide codant pour un polypeptide selon l'invention est inséré dans une cassette d'expression en utilisant des techniques de clonage bien connues de l'homme du métier. Cette cassette d'expression comprend les éléments nécessaires à la transcription et à la traduction des séquences codant pour les polypeptides selon l'invention.

Avantageusement, cette cassette d'expression comprend à la fois des éléments permettant de faire produire un polypeptide par une cellule hôte et des éléments nécessaires à la régulation de cette expression.

Ces cassettes d'expression comprennent dans le sens de la transcription:
- un promoteur fonctionnel dans un organisme hôte;
- un polynucléotide selon l'invention; et
- une séquence terminatrice dans le même organisme hôte.

Tout type de séquence promotrice peut être utilisée dans les cassettes d'expression selon l'invention. Le choix du promoteur dépendra notamment de l'organisme hôte choisi pour l'expression du gène d'intérêt. Certains promoteurs permettent une expression constitutive alors que d'autres promoteurs sont au contraire inductibles. Parmi les promoteurs fonctionnels dans les champignons, on citera notamment celui de glyceraldehyde-3-phosphate deshydrogenase *d'Aspergillus nidulans* (Roberts et al., Current Genet. 15:177-180, 1989). Parmi les promoteurs fonctionnels dans les bactéries, on citera notamment celui de la RNA polymérase du bacteriophage T7 (Studier et al., Methods in enzymology 185:60-89, 1990). Parmi les promoteurs fonctionnels dans les levures, on citera notamment celui du gène Gal1 (Elledge et al., Proc Natl Acad Sciences, USA. 88:1731-1735, 1991) ou les promoteurs GAL4 et ADH de *S.cerevisiae.* Tous ces promoteurs sont décrits dans la littérature et bien connus de l'homme du métier.

Pour l'expression dans *Penicillium funiculosum,* on choisira par exemple des cassettes d'expression comprenant un promoteur histone H4.B, un promoteur acide aspartyl protéase ou un promoteur csl13 (WO 00/68401).

Pour l'expresssion dans la levure *Pichia pastoris,* on choisira par exemple des cassettes d'expression comprenant le promoteur AOX1 inductible par le méthanol (Tschopp, J. F., Sverlow, G., Kosson, R., Craig, W. and Grinna, L. (1987) High-level secretion of glycosylated invertase in the methylotrophic yeast, Pichia pastoris. Biotechnology 5, 1305-1308) ou le promoteur constitutif fort GAP (Waterham, H. R., Digan, M. E., Koutz, P. J., Lair, S. V. and Cregg, J. M. (1997) Isolation of the Pichia pastoris glyceraldehyde-3-phosphate dehydrogenase gene and regulation and use of its promoter. Gene 186, 37-44).

Pour l'expression dans *Schizosacchromyces pombe,* on choisira par exemple des cassettes d'expression comprenant le promoteur de régulation *Nmt1* reprimé par la thiamine et actictivé en abscence de thiamine (Maundrell, K., (1989) Nmt1 of fission yeast. A highly transcribed gene completely repressed by thiamine. J. Biol. Chem. 265, 10857-10864.)

Les cassettes d'expression, selon la présente invention, peuvent en outre inclure toute autre séquence nécessaire à l'expression des polypeptides ou des polynucléotides comme par exemple des éléments de régulation ou des séquences signal permettant la sécrétion des polypeptides produits par l'organisme hôte. On peut notamment utiliser toute séquence de régulation permettant d'augmenter le niveau d'expression de la séquence codante insérée dans la cassette d'expression. Selon l'invention, on peut notamment utiliser, en association avec la séquence de régulation promotrice, d'autres séquences de régulation, qui sont situées entre le promoteur et la séquence codante, telles que des activateurs de transcription ("enhancer").

En outre, les cassettes d'expression, selon la présente invention, peuvent inclure toute autre séquence nécessaire à la sécrétion des polypeptides produits par l'organisme hôte telles que des séquences signal. Pour la sécrétion par *Pichia pastoris,* on peut par exemple utiliser la séquence du facteur α comme signal de sécrétion.

Une grande variété de séquences terminatrices sont utilisables dans les cassettes d'expression selon l'invention, ces séquences permettent la terminaison de la transcription et la polyadénylation de l'ARNm. Toute séquence terminatrice fonctionnelle dans l'organisme hôte sélectionné peut être utilisée.

Pour l'expression dans *Penicillium funiculosum,* on choisira par exemple des cassettes d'expression comprenant un terminateur histone H4.B, un terminateur acide aspartyl protease ou un terminateur csl13 (WO 00/68401).

La présente invention a également pour objet un polynucléotide comprenant une cassette d'expression selon l'invention, avantageusement les cassettes d'expression selon la présente invention sont insérées dans un vecteur.

### Vecteurs

La présente invention concerne donc également des vecteurs de réplication ou d'expression pour la transformation d'un organisme hôte comprenant au moins un polynucléotide ou une cassette d'expression selon la présente invention. Ce vecteur peut notamment être constitué par un plasmide, un cosmide, un bactériophage ou un virus dans lequel est inséré un polynucléotide ou une cassette d'expression selon l'invention. Les techniques de construction de ces vecteurs et d'insertion d'un polynucléotide de l'invention dans ces vecteurs sont bien connues de l'homme du métier. De manière générale, tout vecteur capable de se maintenir, de s'autorépliquer ou de se propager dans une cellule hôte et notamment afin d'induire l'expression d'un polynucléotide ou d'un polypeptide peut être utilisé. L'homme du métier choisira les vecteurs appropriés notamment en fonction de l'organisme hôte à transformer et en fonction de la technique de transformation mise en oeuvre.

Les vecteurs de la présente invention sont notamment utilisés pour transformer un organisme hôte en vue de la réplication du vecteur et/ou de l'expression d'un polypeptide selon l'invention dans l'organisme hôte.

L'invention concerne aussi une méthode pour préparer un polypeptide selon l'invention comprenant les étapes suivantes:
- on transforme un organisme hôte avec un vecteur d'expression comprenant une cassette d'expression selon l'invention et/ou avec un polynucléotide selon l'invention,
- on isole les polypeptides produits par l'organisme hôte.

### Organismes hôtes

La présente invention a également pour objet, un procédé de transformation d'un organisme hôte par intégration dans ledit organisme hôte d'au moins un polynucléotide ou d'une cassette d'expression ou d'un vecteur selon l'invention. Le polynucléotide peut être intégré dans le génome de l'organisme hôte ou se repliquer de manière stable dans l'organisme hôte. Les méthodes de transformation des organismes hôtes sont bien connus de l'homme du métier et largement décrits dans la littérature.

La présente invention concerne également un organisme hôte transformé avec un polynucléotide, une cassette d'expression ou un vecteur selon l'invention.

Par "organisme hôte", on entend Pichia pastaris.

Dans un mode de réalisation préféré de l'invention, l'organisme hôte est une souche de *Pichia pastoris* dans laquelle on exprime une phytase selon l'invention.

Selon la présente invention, l'organisme hôte est transformé avec le polynucléotide choisi parmi les polynucléotides suivants :
a) le polynucléotide dont la séquence est comprise entre la position 1538 et la position 2923 de la SEQ ID No. 1,
b) un polynucléotide codant pour un polypeptide choisi parmi les polypeptides suivants :
   - le polypeptide de la SEQ ID No. 2 ,
   - un fragment du polypeptide de la SEQ ID No. 2 ayant une activité phytase,
   - un polypeptide ayant une activité phytase et présentant au moins 90 % d'identité avec le polypeptide de la SEQ ID No. 2.

Bien qu'il se peut que l'organisme hôte à l'état sauvage possède le polynucléotide ci-dessus défini, la présente invention a pour objet l'organisme hôte transformé. Selon un mode de réalisation de la présente invention, l'organisme hôte est transformé avec un polynucléotide de l'invention, en vue de l'expression du polypeptide de l'invention. Selon un autre mode de réalisation de la présente invention, l'organsime hôte est transformé avec un polynucléotide de l'invention, en vue de la surexpression du polypeptide de l'invention.

Les techniques de construction de vecteurs, de transformation d'organismes hôtes et d'expression de protéines hétérologues dans ces organismes sont largement décrites dans la littérature (Ausubel F.M. et al., "Current Protocols in Molecular Biology" Volumes 1 et 2, Greene Publishing Associates et Wiley -Interscience, 1989; T.Maniatis, E.F.Fritsch, J.Sambrook, Molecular Cloning A laboratory Handbook, 1982).

### Additifs alimentaires et aliments pour animaux

La présente invention concerne également des additifs alimentaires apportant une activité phytase. L'apport de ce type d'activité enzymatique permet d'améliorer la digestibilité de l'aliment et d'améliorer sa valeur nutritionnelle.

On entend par additif nutritionnel une substance ajoutée intentionnellement à un aliment, généralement en petites quantités, pour améliorer ses caractéristiques nutritionnelles ou sa digestibilité. Les additifs nutritionnels pour animaux peuvent par exemple contenir des vitamines, des sels minéraux, des acides aminés et des enzymes.

Typiquement, les additifs nutritionnels pour animaux comprennent un polypeptide selon l'invention, un organisme hôte selon l'invention ou un surnageant de culture/moût de fermentation d'un organisme hôte selon l'invention. Ainsi, les polypeptides ayant une activité phytase selon l'invention peuvent être purifiés ou isolés d'une souche de *Debaryomyces castellii* ou d'un organisme hôte recombinant pour la fabrication d'un additif nutritionnel pour animaux. Alternativement, une souche de *Debaryomyces castellii* ou un organisme hôte produisant des phytases selon l'invention peuvent être utilisés directement pour la fabrication d'un additif nutritionnel pour animaux. Dans un mode de réalisation préféré de l'invention, le surnageant de culture ou moût de fermentation d'un organisme hôte selon l'invention est utilisé pour la fabrication d'additifs nutritionnels pour animaux. Ce mode de réalisation est particulièrement avantageux lorsque la phytase est secrétée dans le milieu extracellulaire par organisme hôte. Habituellement, ce surnageant de culture est concentré ou lyophilisé pour la fabrication de l'additif nutritionnel.

Ainsi, l'invention concerne aussi un procédé de préparation de phytase comprenant les étapes suivantes :
a) mise en culture d'un organisme hôte transformé selon l'invention dans des conditions d'induction de l'expression de la phytase,
b) séparation du surnageant de culture comprenant la phytase.

Ce surnageant de culture ou moût de fermentation peut ensuite être concentré ou lyophilisé pour la formulation d'un additif alimentaire ou d'un aliment pour animaux. Le procédé peut comprendre des étapes supplémentaires de purification de la phytase à partir du surnageant de culture.

Si l'organisme hôte ne secrète pas la phytase dans le milieu de culture, une étape supplémentaire de cassage des cellules et de purification de l'extrait cellulaire peut être nécessaire.

Les additifs nutritionnels de la présente invention comprennent au moins une phytase selon l'invention mais peuvent également comprendre d'autres substances nutritionnelles comme des vitamines, des acides aminés ou des sels minéraux.

Les additifs selon l'invention accroissent la digestibilité des aliments, contribuant ainsi à une meilleure valorisation nutritionnelle des régimes à base de céréales (blé, orge, maïs, avoine, seigle, ...) et de tourteaux oléagineux (soja, tournesol, colza, ...) notamment.

La présente invention concerne aussi les aliments pour animaux comprenant une base nutritionnelle et un additif nutritionnel selon l'invention. Ces aliments se présentent habituellement sous la forme de farines ou de granulés dans lesquels sont incorporés les additifs selon l'invention.

La présente invention a également pour objet des aliments pour animaux comprennent un polypeptide selon l'invention, un organisme hôte selon l'invention où un moût de fermentation/surnageant de culture d'un organisme hôte selon l'invention.

On entend par aliment tout ce qui peut servir à la nourriture des animaux.

Dans un mode de réalisation de l'invention, les additifs nutritionnels et les aliments pour animaux selon l'invention comprennent une association d'au moins deux phytases ayant des activités complémentaires. Ces additifs et ces aliments comprennent ainsi au moins une phytase selon l'invention associée à un autre phytase. La phytase associée à la phytase selon l'invention peut être par exemple choisie parmi les phytases des organismes suivants : *Schwanniomyces occidentalis, Aspergillus awamori* (phytase A et phytase B), *Aspergillus niger* (phytase A et phytase B), *Penicillium funiculosum, Aspergillus oryzae, Peniophora lycii, Aspergillus ficuum, Aspergillus nidulans, Talaromyces thermophilus, Aspergillus fumigatus* et *Aspergillus terreus.*

De façon avantageuse, la phytase selon la présente invention hydrolyse tous les groupements phosphate de l'acide phytique. Elle peut donc notamment être utilisée pour améliorer ou compléter l'activité de phytases qui n'hydrolysent pas tous les groupements phosphates. De préférence, la phytase selon la présente invention est associée à la phytase *d'Aspergillus niger* (Ullah, A. H. J and Sethumadhavan, K., (2003) PhyA gene product of Aspergillus ficuum and Peniophora lycii produces dissimilar phytases. Biochemical and Biophysical Research Communications 303 : 463-468) ou à une phytase d'une souche de *Penicillium funiculosum* (WO 03/054199, WO 99/57325).

Préférentiellement, les additifs et les aliments comprennent une phytase selon la présente invention associée à une phytase *d'Aspergillus niger* ou à une phytase de *Penicillium funiculosum.*

Pour l'élevage intensif des animaux, les aliments pour animaux comprennent habituellement une base nutritionnelle et des additifs nutritionnels.

On entend par base nutritionnelle, ce qui constitue l'essentiel de la ration alimentaire de l'animal, constitué à titre d'exemple par un mélange de céréales, de protéines et de matières grasses d'origine animale et/ou végétale.

Les bases nutritionnelles pour animaux sont adaptées à l'alimentation de ces animaux et sont bien connues de l'homme du métier. Habituellement, ces bases nutritionnelles comprennent par exemple du maïs, du blé, du pois et du soja. Ces bases nutritionnelles sont adaptées aux besoins des différentes espèces animales auxquelles elles sont destinées. Ces bases nutritionnelles peuvent déjà contenir des additifs nutritionnels comme des vitamines, des sels minéraux et des acides aminés.

Dans un mode de réalisation préféré, l'invention concerne des aliments pour animaux monogastriques et notamment pour les volailles et les porcs. Les volailles comprennent notamment les poules pondeuses, les poulets de chair, les dindes et les canards. Les porcs comprennent notamment les porcs croissance et finition ainsi que les porcelets.

### Description des figures

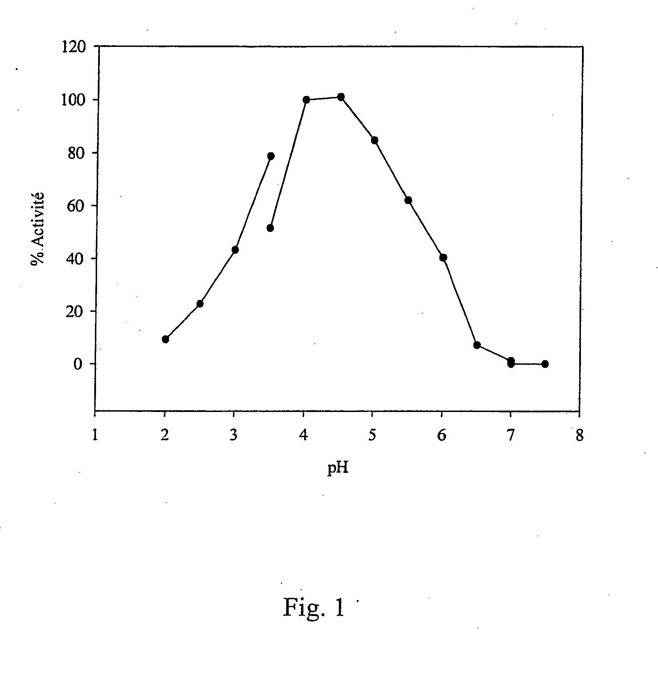

**Figure 1** : Activité de la phytase de *D. castellii* en fonction du pH (pH 3 à 3,5 Tampon glycine 200 mM, pH 3,5 à 7 Tampon acétate de sodium 200 mM, pH 7 à 7,5 Tampon tris HCl 200 mM). Les différentes valeurs obtenues à pH 3,5 avec 2 tampons différents, montre l'effet de la nature du tampon sur l'activité phytasique.

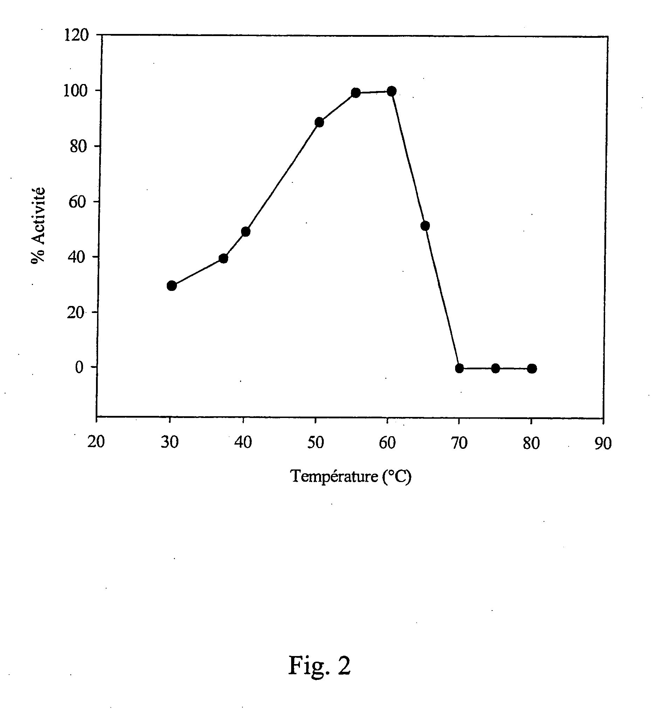

**Figure 2** : Activité de la phytase de *D. castellii* en fonction de la température. L'activité est mesurée à pH 4, à 37°C pendant 20 minutes.

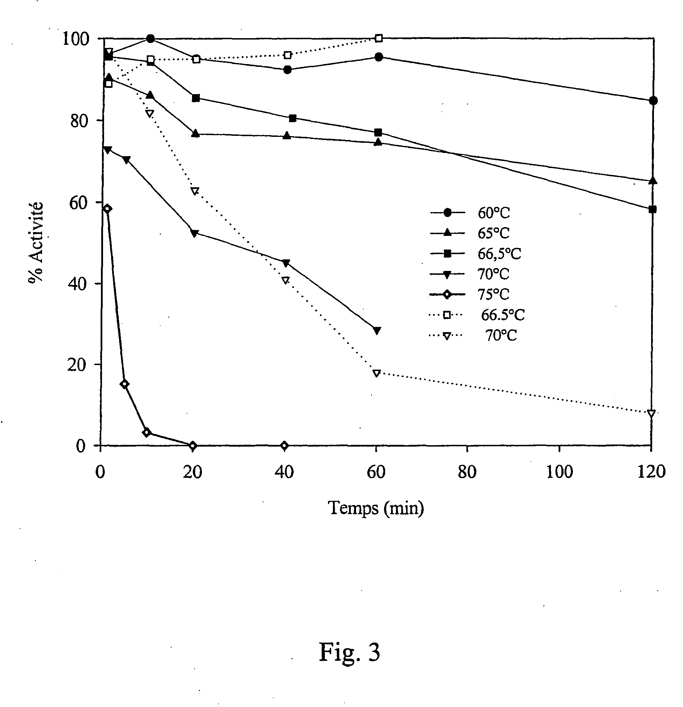

**Figure 3** : Dénaturation thermique de la phytase de *D. castellii.* L'extrait est pré incubé à différentes températures pendant 1 - 10 - 20 - 40 - 60 - 120 minutes soit dans de l'eau (lignes pleines), soit dans du tampon acétate 125 mM, pH4 (lignes pointillées). L'activité est ensuite mesurée à 37°C pendant 20 min.

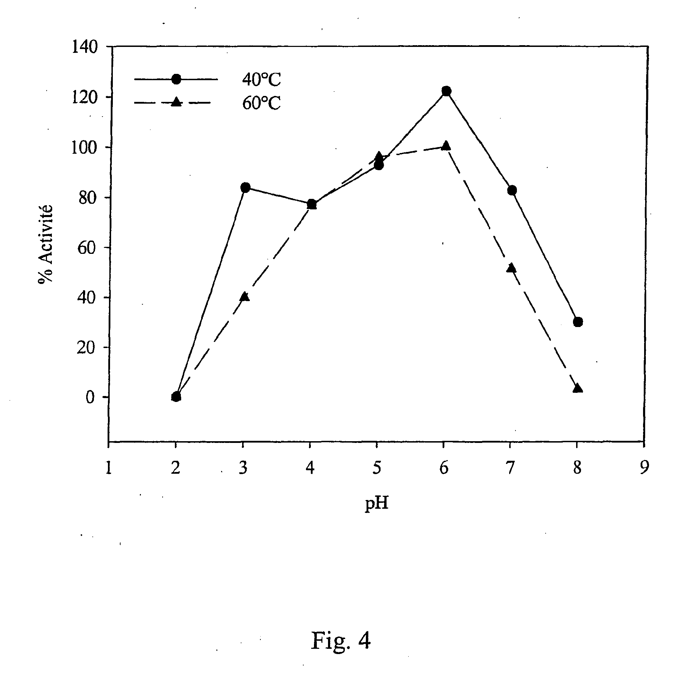

**Figure 4** : Etude de la stabilité au pH et à la température. L'activité phytasique de *D. castellii* est mesurée après traitement de l'extrait à différents pH pendant 1 heure à deux température (40°C ou 60°C). L'activité est mesurée à pH 4.

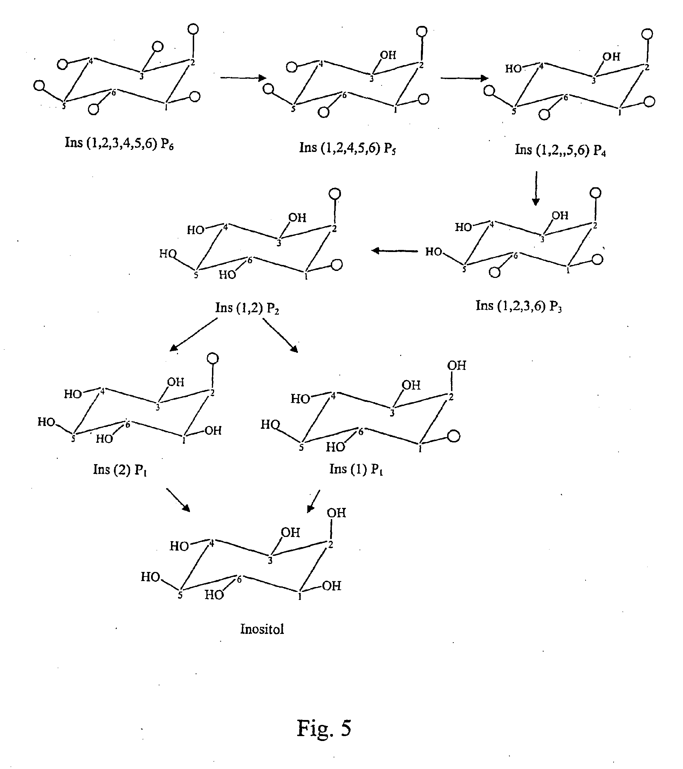

**Figure 5** : Schéma d'hydrolyse de l'acide phytique par la phytase de D. *castellii.*

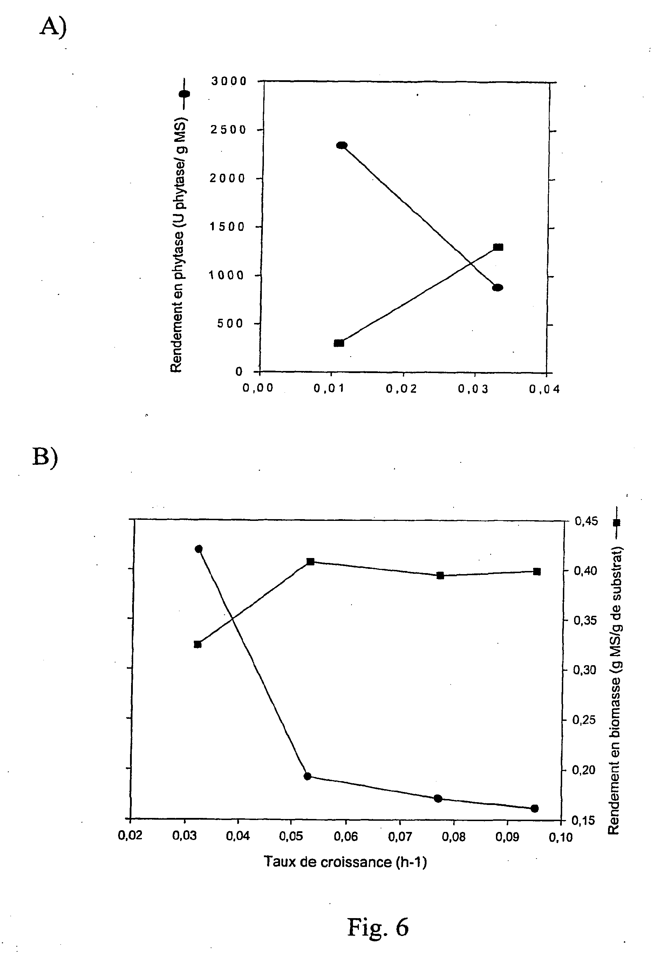

**Figure 6** : Evolution du rendement en phytase recombinante (cercles) et en biomasse (carrés) en fonction du taux de croissance au cours des cultures en mode fed-batch (A) et continu (B).

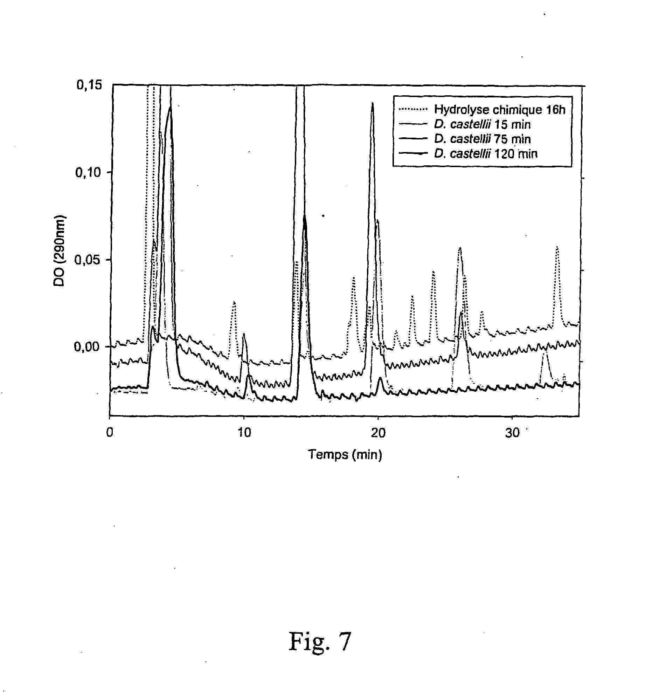

**Figure 7** : Suivi de l'apparition des divers inositols phosphates par chromatographie après l'hydrolyse chimique et hydrolyse enzymatique de l'Ins P₆ par la phytase de *D. castellii* après 15, 75, 120 min d'hydrolyse.

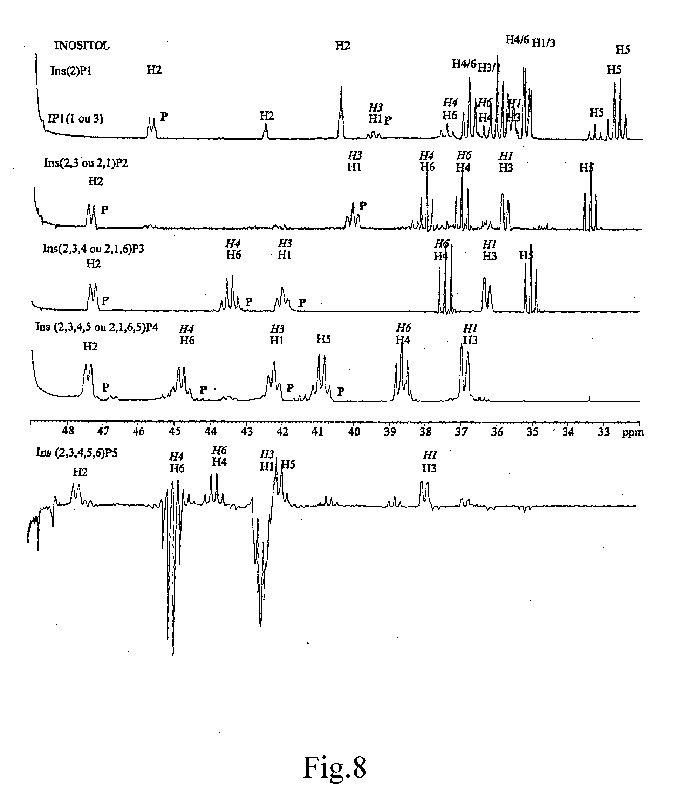

**Figure 8** : Assemblage et comparaison des spectres des 4 fractions d'hydrolyses contenant les InsP1, InsP2, InsP3 et InsP4. Les attributions alternatives sont en italiques. Afin de compléter cette comparaison, sous l'échelle des déplacements chimiques, le spectre de l'InsP5 obtenu par différence en debut de cinétique est également représenté. Les signaux positifs sont ceux de l'InsP5 et les deux signaux négatifs correspondent à l'InsP6 de départ. Ces spectres, caractéristiques des différents inositols phosphates permettent de suivre l'apparition et la disparition des différents produits au cours de la cinétique d'hydrolyse.

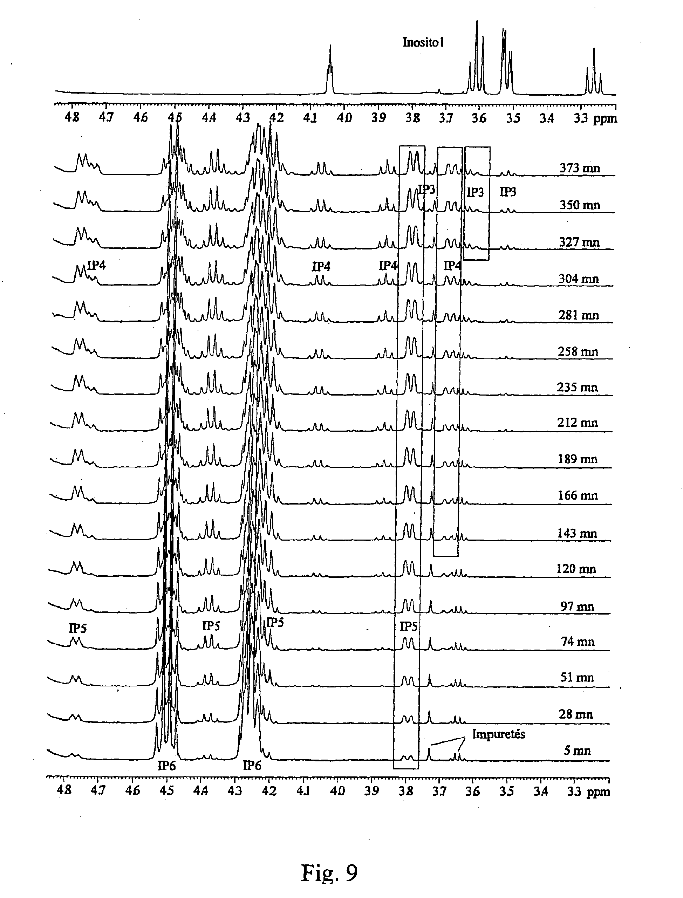

**Figure 9** : Cinétique lente suivie à 500 MHz et à 17°C avec une solution de 1.7 mM d'InsP6, 10 mM de tampon acétate, pH 4.0 (500 µl H2O/D2O, 16/84 v/v) et 1µl de la solution d'enzyme. L'intervalle de temps entre deux spectres est de 23 minutes. Après une nuit 20 µl d'enzyme ont été ajoutés pour accélérer la réaction. Après 16 H l'hydrolyse est complète et le spectre caractéristique de l'inositol est obtenu (spectre du haut). Cette cinétique montre clairement l'apparition successive de l'InsP5, de l'InsP4 et de l'InsP3

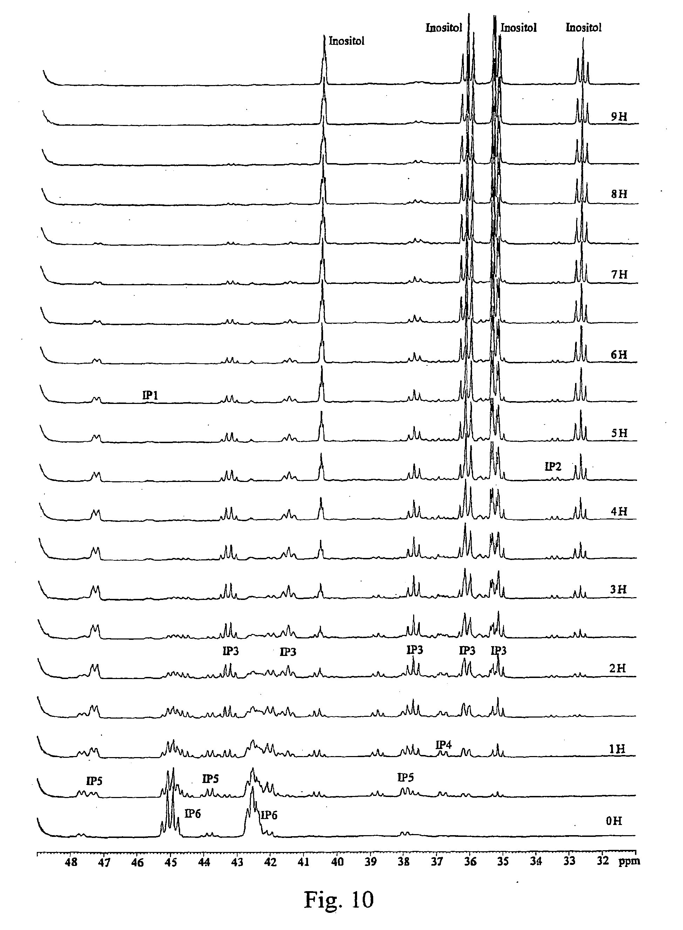

**Figure 10** : Cinétique plus rapide permettant un suivi de l'hydrolyse complète de l'InsP6 (600 MHz, 17 °C, 500 µl D2O, 3,8 mM en InsP6 pH 4.0, 5µl d'enzyme). Un spectre a été enregistré toutes les 3 mn pendant 20H (400 spectres). Après 10 heures, l'hydrolyse est pratiquement complète. Les spectres tracés correspondent à un spectre sur 10 (30 mn entre deux spectres). Quelques signaux caractéristiques sont annotés.

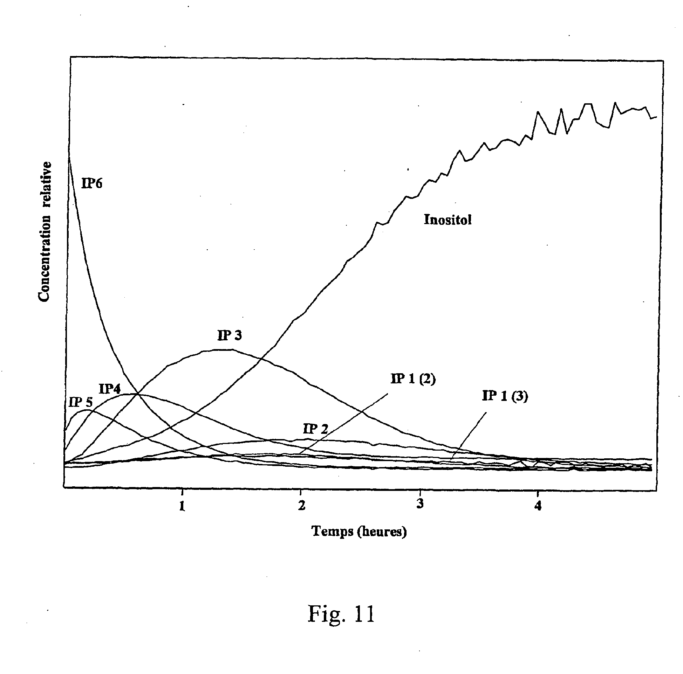

**Figure 11** : Evolution des concentrations sur 5 heures des différents produits au cours de l'hydrolyse. Ce graphe permet de connaître à quel temps la concentration optimale pour chacun des produits est atteinte. Remarque: La hauteur du signal RMN caractéristique de chacun des composés a été utilisée pour évaluer les variations de concentrations. Les signaux utilisés ne représentent pas toujours le même nombre de protons et n'ont pas toujours la même multiplicité. De ce fait, les concentrations ne peuvent pas être comparées entre elles.

### Exemples

### Exemple 1 : Production, caractérisation biochimique et stéréospécificité de la phytase de Debaryomyces castellii

### MATERIELS ET METHODES

### 1. Organisme

La souche utilisée est répertoriée au Centraal bureau voor Schimmelculture (Delft) sous le nom *Debaryomyces castellii* CBS 2923.

### 2. Milieux et conditions de culture

*Milieu synthétique pour les cultures en Batch* (MSA-B): Glucose (10 g/L) ; Phytate de sodium C₆H₆O₂₄P₆Na₁₂ (0,4 g/L)
Sels minéraux : (NH₄)₂SO₄ (3g/L), MnSO₄,H₂O (7,5 mg/L), KCl (0,5 g/L), MgSO₄,7H₂O (0,5 g/L), CaCl₂,2H₂O ( 0,1 g/L)
Oligo-éléments : H₃BO₄ (500 µg/L), CuSO₄,5H₂O (40 µg/L), Kl (100 µg/L), Na₂MoO₄,2H₂O (200 µg/L), ZnSO₄,7H₂O (400 µg/L), FeCl₃,6H₂O (200 µg/L).
Vitamines : Pantothénate Ca (2 mg/L), Thyamine (B1) (2 mg/L), Myo-inositol (2 mg/L), Pyridoxine (B6) (2 mg/L), Ac. Nicotinique (PP) (0,5 mg/L), Biotine (0,02 mg/L).
*Milieu synthétique pour les cultures, en continue* (MSA-C) : Composition du milieu MSA-B les divers composants sont 10 fois plus concentrés.

### Culture en Erlenmeyer

La première préculture est réalisée en présence de YMPG (glucose 10 g/L, yeast extract 3 g/L, bacto peptone 5 g/L, malt extract 3 g/L). Les cultures sont réalisées dans des fioles d'Erlenmeyer remplies au 1/10^{ème} de leur volume en présence de milieu MSA-B tamponné à pH 5,4 avec du tampon tartrate 0,2 M. Elles sont conduites en milieu aéré sur agitateur va-et-vient (80 oscillations par minute, amplitude 7 cm), à 28°C.

### Culture en fermenteur

Les cultures sont réalisées dans un fermenteur Applikon (The Netherlands) (1,5 L de volume utile) ou Braun Biostat E (3 L de volume utile). Le pH est mesuré par une sonde Ingold. Il est régulé par ajout de soude 2 M ou d'acide sulfurique 1 M. L'aération est assurée par insufflation de 2 v.v.m (volume d'air.(volume de culture)⁻¹.(minute)⁻¹) d'air stérilisé par filtration. La pression partielle en oxygène dissous est mesurée à l'aide d'une sonde polarographique Ingold. Elle est maintenue à une valeur supérieure à 30% par variation de la vitesse d'agitation. La température est maintenue à 28°C. Le pilotage et l'acquisition des données sont effectués en ligne à l'aide d'un logiciel d'acquisition Bioexpert (Applikon).

### Analyse des gaz en sortie de fermenteur

La concentration en CO₂ dans les gaz effluents est mesurée à l'aide d'un analyseur à Infrarouge Beckman Industrial 870. La concentration en O₂ est mesurée à l'aide d'un analyseur Beckman Industrial 775A dont le détecteur utilise la susceptibilité paramagnétique de l'oxygène moléculaire.

### Dosage des substrats carbonés au cours de la culture

Les substrats et les métabolites présents dans le milieu de culture (Glucose, acétate, éthanol) sont séparés et quantifiés par Chromatographie Liquide Haute Performance (CLHP) à l'aide d'une colonne FFJ (Waters) d'exclusion d'ions. La phase mobile est de l'acide phosphorique 3 mM, dont le débit est de 1 ml/min. Un échantillon du milieu de culture est prélevé en continu toutes les deux heures et filtré stérilement de façon tangentielle (filtre Millipore 0,22 µm GV) à l'aide du module de filtration A-SEP Applikon et du module FAM d'acquisition et filtration Waters. Les substrats carbonés sont détectés par réfractométrie (Waters 410). L'ensemble est piloté par le système de contrôle Waters 600 E. Les chromatogrammes sont analysés grâce au logiciel Millenium (Waters).

Des gammes étalons ont été réalisées pour chaque substrat sur une échelle allant de 1 à 50 g/L

### 3. Purification

### Ultrafiltration

Le surnageant de culture obtenu après centrifugation est filtré sur une membrane de seuil de coupure 0,22 µm (Millipore) avant d'être ultrafiltré sur cassette d'ultrafiltration à flux tangentiel Filtron (surface : 836 cm²) dont le seuil d'exclusion est de 10 kDa. Le concentrât est lavé 3 fois (VN) avec de l'eau ultra pure puis concentré par un facteur 25. L'extrait obtenu est utilisé pour la purification.

### Chromatographie hydrophobe

La séparation des protéines est réalisée à 20°C sur une colonne HiPrep 16/10 Phenyl FF (Amersham) de diamètre interne 16 mm et de longueur 100 mm (volume 20 mL).

Avant l'injection sur le gel de purification, les échantillons sont équilibrés dans du sulfate d'ammonium 2 M. Le mélange est laissé, 2 à 16 heures, à 4°C puis centrifugé (12000 g, 20 min) afin d'éliminer les protéines précipitées. Le surnageant de la centrifugation constitue l'extrait déposé sur le gel.

Le gel est tout d'abord équilibré avec l'équivalent de 5 volumes de colonne à l'aide d'une solution tampon Tris-HCl 50 mM, pH 6,1 et de sulfate d'ammonium 2 M. 1 à 5 mL d'échantillon sont injectés.

Les protéines non fixées sont éliminées par un lavage équivalent à 5 volumes de colonne avec la solution tampon sulfate d'ammonium d'équilibration.

L'élution est réalisée en effectuant un gradient de trois segments linéaires : (1) sulfate d'ammonium de 2 à 1,7 M, sur une durée équivalente à 1,5 volumes de colonne, (2) sulfate d'ammonium 1,7 M, sur une durée équivalente à 4 volumes de colonne, (3) sulfate d'ammonium de 1,7 à 0 M, sur une durée équivalente à 0,1 volumes de colonne. La phytase est éluée à 1,7 M de sulfate d'ammonium.

Le débit est fixé à 5 mL/min. Des fractions de 4 mL sont récoltées en sortie de colonne, l'absorbance est mesurée à 280 nm.

Les fractions actives sont rassemblées, lavées contre de l'eau ultra pure et concentrées par ultrafiltration (membrane Millipore, seuil de coupure 10 kDa).

### 4. Electrophorèse

Les électrophorèses en conditions dénaturantes et non dénaturantes sont réalisées sur des gels précoulés de 4 à 15% d'acrylamide (Biorad). Les protéines sont détectées au bleu de Coomassie.

La révélation spécifique des phytases est réalisées en incubant les gels dans 100 ml d'une solution tampon acétate de sodium 250 mM pH 5,5 renfermant 200 mg d' α naphtyl P (Sigma) et 100 mg de fast Garnet GBC (Sigma) et 92 mg de phytate de sodium. Après hydrolyse de l'α naphtyl P, il se forme un complexe α naphtyl/Fast Garnet GBC de couleur brune.

### 5. Digestion à l'endoglycosidase H

Déglycosylation : 1000 unités d'endoglycosidase H (Biolab Ozyme P0702S) sont ajoutées à l'échantillon dénaturé renfermant environ 20 µg de protéines. Le mélange est mis à incuber 2 heures à 37°C au bain-marie.

### 6. Détermination du poids moléculaire par spectrométrie de masse

L'analyse est réalisée sur la phytase purifiée sur SDS-PAGE à l'aide d'un spectromètre MALDL-TOF Biflex III scout 384 (Bruker, Breme, Germany).

### 7. Méthodes analytiques

### 7.1 Détermination de la matière sèche

La concentration cellulaire est obtenue par mesure de la densité optique (DO) à l'aide d'un spectrophotomètre Beckman DU530. Une unité de DO correspond à 0,570 g/L de biomasse.

### 7.2 Dosage des protéines

La teneur en protéines est déterminée par la méthode de Bradford (Bradford, M. (1976) A rapid and sensitive method for the quantification of microgram quantities of proteins utilizing the principle of protein-dye binding. Anal. Biochem. 72, 248-254). (Biorad Protein Assay Dye Reagent Concentrate, BIORAD 500-0006), l'absorbance est mesurée à 595 nm (spectrophotomètre UV/visible Beckman DU 530). L'étalonnage est effectué avec une gamme de sérum albumine bovine.

### 7.3 Méthode enzymatique

L'activité phytasique est mesurée en suivant la libération de phosphate inorganique au cours du temps.

L'activité est mesurée en présence de 8 mM de phytate de sodium (Sigma) dissous dans un tampon acétate de sodium pH 5,5 ou pH 4, 250 mM, 1 mM de CaCl₂, à 37°C (5 volumes). La réaction est déclenchée par l'addition de l'extrait enzymatique (1 volume). La réaction est stoppée par acidification du milieu par de l'acide trichloracétique 20% (1 volume de milieu réactionnel + 1 volume d'acide). La quantité de phosphate libéré est déterminée après différents temps d'incubation.

Une unité enzymatique (U) est définie comme la quantité d'enzyme qui libère une µmole de phosphate inorganique en une minute.

### Caractérisation de l'enzyme

L'effet du pH sur l'activité phytasique est déterminé en utilisant les solutions tampon suivantes : Glycine-HCl 200 mM, pH 2-3.5; Acétate de sodium-acide acétique 200 mM, pH 3.5-7; et Tris-HCl 200 mM, pH 7-9. Les réactions sont réalisées à +37°C en utilisant le phytate comme substrat. La température optimale est déterminée en faisant varier la température de +30 à +80°C. Les réactions sont réalisées à pH 4 (tampon acétate de sodium 200 mM) en utilisant le phytate comme substrat. La stabilité thermique est déterminée par incubation de l'échantillon enzymatique dans du tampon acétate de sodium 125 mM, pH 4, pendant différentes durées à des températures allant de +37 à +70°C. Après le traitement thermique, le mélange est refroidi dans de la glace et l'activité phytasique est déterminée en utilisant le phytate comme substrat. Les paramètres cinétiques sont déterminés à +37°C et à pH 4 pour les expériences avec le phytate de sodium et à pH 5.5 pour les expériences avec le p-NPP.

### 7.4 Dosage des phosphates

La quantité de phosphate libéré est mesurée par colorimétrie. La solution de révélation, préparée extemporanément, contient du sulfate de fer (380 mM, 1 volume) et de l'heptamolybdate d'ammonium (12 mM, 4 volumes). La mesure de l'absorbance, à 700 nm, se fait après 30 minutes de révélation à température ambiante (1 volume de milieu réactionnel + 1 volume de solution de révélation), à l'aide d'un spectrophotomètre UV/visible (Beckman DU 530).

Une droite d'étalonnage est préalablement établie avec du potassium dihydrogénophosphate.

### 7.5 Conditions d'hydrolyse des phytases pour l'étude de la stéréospécificité

Le tube réactionnel contient 2 volumes de phytate de sodium (20 mM), 2 volumes de tampon acétate pH 4 (0,25 M) et 1 volume d'enzyme diluée (0,6 U/mL en final).

Des prélèvements sont réalisés à différents temps pendant 6 heures. La réaction est arrêtée par chauffage à 100°C (10 minutes).

### 7.6 Détermination des inositols phosphates

### 7.6.1 Par HPI C

Il s'agit d'une méthode utilisant la chromatographie ionique à haute performance (HPIC) pour séparer et déterminer les inositols mono- à hexaphosphates obtenus par la dégradation de l'acide phytique par les phytases étudiées (Hatzack, F., Hübel, F., Zhang, W., Hansen, P.E. and Rasmussen, S.K. (2001) Inositol phosphates from barley low-phytate grain mutants analysed by metal-dye detection HPLC and NMR. Biochem. J. 354, 473-480 ; Skoglund, E., Carlsson, N.G. and Sanberg, A.S. (1997) Determination of isomers of inositol mono- to hexaphophates in selected foods and intestinal contents using High-Performance lon Chromatography. J. Agric. Food Chem. 45, 431-436 ; Türk, M., Sandberg, A.S., Carlsson, N.G. and Andlid, T. (2000) Inositol hexaphosphate hydrolysis by baker's yeast. Capacity, kinetics, and degradation products. J. Agric. Food Chem. 48, 100-104). La méthode inclue la séparation des différents Ins Pₙ sur colonne échangeuse d'ions en HPLC avec un gradient d'élution et la réaction post colonne pendant laquelle les inositols phosphates se complexent avec du fer et sont détectés par UV à 290 nm. Ce système permet de détecter des Ins P₂ aux Ins P₆ mais seuls les différents isomères d'Ins P₄ et Ins P₅ peuvent être séparés.

### Préparation d'un échantillon de référence.

Les pics sont identifiés après une hydrolyse chimique. 50 mg de phytate de sodium sont mis dans 5 mL de HCl (6 M) à 100°C pendant 16 heures. Des aliquotes de 25 µL sont séchées au Speed Vac puis reprises dans 100 µL d'HCl 0,025 M pour avoir environ 300 nmoles par injection.

### Analyse des échantillons.

La séparation des différents pics est réalisée par chromatographie d'échange d'anions forts sur une colonne analytique Omni Pac PAX-100 (4 x 250 mm) et une précolonne de garde PAX-100 (4 x 50 mm) (Dionex Corp., Sunnyvale, CA). Le débit est de 0,8 mL/min, la boucle d'injection de 100 µl. Les Ins Pₙ sont élués avec un gradient de 5-98% HCl (0,5 M) en conjugaison avec de l'eau ultra pure et un solvant organique (50% de 2-propanol). Les éluants sont combinés selon le **tableau 1** suivant :

| Temps (min) | HCl (%) | 2- propanol (%) | Eau (%) |
|---|---|---|---|
| T (0) | 5 | 2 | 93 |
| T (40) | 98 | 2 | 0 |
| T (45) | 5 | 2 | 93 |

Un temps de 15 minutes est nécessaire pour équilibrer la colonne après chaque chromatographie.

Les inositols phosphates sont détectés après une réaction post colonne par mesure de l'absorbance à 290 nm à l'aide d'un spectrophotomètre UV (Biocad, Sprint). L'éluant est mélangé, dans une réaction post colonne, avec 0,1% de Fe(NO₃)₃.9H₂O dans une solution à 2% de HCIO₄. Le débit de la pompe du réactif (Minipuls 3, Gilson) est de 0,4 mL/min. Le passage dans un serpentin en Teflon (0,25 mm, 4 m) permet de complexer les Ins Pₙ et le fer pour la détection (Phillippy, B.Q. and Bland, J.M. (1988) Gradient ion chromatography of inositol phosphates. Anal. Biochem. 175, 162-166).

### 7. 6.2 Par RMN

Après lyophilisation, les différents échantillons d'inositols isolés par HPIC (50-200 µg) sont solubilisés dans 500 µl de D2O.

Les spectres protons ont été enregistrés à 500 ou 600 MHz sur des spectromètres Avance Bruker équipés d'une cryosonde (1H, 13C et 15N) et de gradients sur l'axe z. Les spectres protons découplés du phosphore et les spectres de corrélation 1H-31P (HMQC) ont été enregistrés sur un spectromètre Avance Bruker 400 MHz avec une sonde TBI. Le signal résiduel de l'eau a été supprimé par une présaturation sélective d'une durée de 1 s. Tous les spectres ont été enregistrés à 17 °C. Les spectres protons sont calibrés par rapport au (trimethylsilyl)-3-propionate de sodium-*d4* (TSP, 0 ppm) ou au signal résiduel de l'eau (4.914 ppm à 17°C). Pour l'attribution des résonances proton, les expériences COSY et TOCSY ont été enregistrées avec 512 incréments de temps. Le temps de contact utilisé pour le TOCSY est de 50 ms. Les expériences HMQC ont été obtenues avec 64 incréments de temps.

L'attribution des spectres protons sera faite par l'analyse des expériences COSY et TOCSY. Il est à noter que pour certains isomères possédant un plan de symétrie, les protons H1 et H3 et les protons H4 et H6 ne peuvent être différenciés. Dans ce cas ils seront notés H1 (ou H3) et H4 (ou H6). Une fois l'attribution obtenue, reste à déterminer les positions phosphorylées. Celles-ci seront déterminées à l'aide de deux expériences différentes, d'une part à l'aide du spectre proton découplé du phosphore et d'autre part par l'expérience HMQC. La comparaison du spectre proton sans et avec découplage du phosphore permet d'identifier les signaux qui possèdent un couplage ³J_{HCOP} 8.5-10Hz et ainsi la (les) position(s) de phosphorylation.

L'expérience ¹H-³¹P HMQC, par l'intermédiaire de la constante de couplage ³J_{HCOP}, quant à elle permet d'identifier les corrélations proton-phosphore. Lorsque l'attribution des résonances protons est connue, les spectres découplés du phosphore et HMQC permettent d'identifier les positions phosphorylées sans ambiguïté. La position des groupements phosphates est également vérifiée par l'analyse des constantes de couplage du spectre proton.

### 7. 6.3 Cinétique d'hydrolyse suivie par RMN

L'échantillon utilisé pour suivre l'hydrolyse par RMN est préparé à partir d'une solution mère d'InsP6/tampon phosphate, 96/700 mM dans D2O. Typiquement, 20 µl de cette solution sont ajoutés à 480 µl de D2O (dilution 25) pour conduire à un échantillon qui contient 3.8 mM d'InsP6 et 28 mM de tampon acétate de sodium et du TSP comme référence interne. En fonction de la vitesse d'hydrolyse souhaitée, une plus ou moins grande quantité d'enzyme (1 à 20 µl de la solution mère à 3 U/ml) est ajoutée. La cinétique est suivie à 17°C par l'enregistrement d'un spectre (32 scans) toutes les 3 minutes pendant 20 heures (400 spectres). A partir des spectres caractéristiques des différents inositols phosphates déterminés précédemment, leur apparition et disparition peuvent être suivies sur l'ensemble de la cinétique.

### RESULTATS

### 1- Etude de la biosynthèse de la phytase de Debaryomyces castellii

La biosynthèse de la phytase a été suivie en culture batch et continue afin de déterminer les conditions optimales de production. Les cultures sont réalisées sur milieu synthétique (cf. Matériels et Méthodes). Une étude préalable nous a permis de déterminer une concentration optimale en phytate de 0,4 g/l pour produire 5 g/l de biomasse. Cette concentration est nécessaire et suffisante pour assurer la croissance cellulaire maximum et ne pas réprimer la biosynthèse des phytases. Des prélèvements sont effectués au cours du temps ; la biomasse, l'activité phytasique sont mesurées.

### 1.1 Production en culture batch

Cinq cultures Batch sont réalisées à pH 3, 4, 5, 6 et 7 en présence de 10 g/L de glucose et de milieu MSA-B. La biomasse maximum est obtenue à pH 5, le taux de croissance est diminué de 20% à pH 3 et à pH 7. L'activité phytasique maximum, mesurée dans les surnageants de culture, est obtenue à pH 4, aucune activité n'est détectée à pH 3. L'activité phytasique augmente d'environ 20%, 12 heures après le début de la phase stationnaire. Il est possible qu'une partie de la phytase soit pariétale et libérée dans le milieu en phase de non croissance. Nous avons vérifié que l'on obtenait un même niveau d'induction en présence ou en absence de sel de calcium.

### 1.2 Production en culture continue

La culture continue est réalisée à pH 4, en présence de 100 g/L de glucose et de milieu MSA-C. Les mesures de biomasse et d'activités phytasiques sont effectuées après au moins 3 renouvellements du fermenteur.

La production de biomasse est constante jusqu'à un taux de dilution de 0,20 h⁻¹, avec un rendement Y_{Biomasse/substrat} (g/g) de 50%, le métabolisme cellulaire est oxydatif, le coefficient respiratoire (QR) est égal à 1. La production de phytase augmente avec le taux de dilution. Pour un taux de dilution (D) de 0,25 h⁻¹, le rendement de croissance diminue de 40%, le QR est supérieur à 1, le métabolisme cellulaire est oxydo-fermentaire, il y a formation de métabolites secondaires tels que de l'acétate et de l'éthanol. La production de phytase diminue également d'un facteur 5.

La meilleure production (1487 U/L) est obtenue à D=0.20 h⁻¹. Il faut noter que 20% de phytase sont liés aux cellules, le maintien de la culture à 4°C pendant au moins 4 heures permet le relargage de l'enzyme dans le milieu de culture.

### 2- Etude de la phytase

### 2.1 Purification

Les différentes étapes de purification de la phytase de *D. castellii* sont résumées dans le **tableau 2.**

**Tableau 2: Purification de la phytase de Debaryomyces castellii par chromatographie hydrophobe.**

| | Activité totale (U) | Protéines Totales (mg) | Activité Spécifique (U/mg) | Facteur de purification | Rendement % |
|---|---|---|---|---|---|
| Extrait brut | 144 | 12.4 | 11,6 | 1 | |
| Extrait concentré et ultrafiltré | 136,5 | 8,2 | 16,7 | 1,45 | 95 |
| Chromatographie hydrophobe | 84,4 | 0,54 | 156 | 13,6 | 59 |

L'extrait brut a une activité spécifique de 11,4 U/ml. Après une étape de concentration et ultrafiltration, l'extrait est purifié par chromatographie hydrophobe. La phytase est purifiée en une seule étape par un facteur 13 avec un rendement de 59%. L'activité spécifique est de 156 U/mg. La présence d'une seule bande protéique sur une électrophorèse SDS-PAGE montre que l'enzyme est pure.

### 2.2 Masse molaire et structure

### 2.2.1 Détermination de la masse molaire par électrophorèse ou chromatographie de gel perméation

Une électrophorèse SDS-PAGE permet d'estimer la masse molaire de la phytase à 77 kDa et de l'enzyme déglycosylée par traitement à l'endoglycosidase H à 51 kDa, soit 34% de glycosylation. En condition non dénaturante la masse molaire de la phytase est plus difficile à déterminer en raison de la présence de traînées, elle se situe entre 440 en 150 kDa. Celle de l'enzyme déglycosylée est de 87 kDa. Une coloration spécifique permet de montrer que la phytase déglycosylée est active.

La masse molaire déterminée par chromatographie de gel perméation (colonne HR 200 Pharmacia) est de 318 kDa pour la phytase glycosylée et de 218 kDa pour la phytase déglycosylée.

### 2.2.2 Spectrométrie de masse

La détermination de la masse par spectrométrie de masse confirme les résultats obtenus par électrophorèse SDS-PAGE, soit 74 kDa pour la phytase et 53 kDa pour l'enzyme déglycosylée soit 28,4 % de glycosylation.

La phytase native serait donc constituée de 4 monomères de masse identique.

### 2.2.3 Cristallographie

Au cours de l'année de priorité, la structure de la phytase de *Debaryomyces castellii* (461 résidus) a été déterminée par cristallographie avec une résolution de 2,3 Å.

C'est un tétramère qui contient 10 molécules de N-acetyl glucosamine et 1256 molécules d'eau.

La structure est accessible sur le site du RCSB Protein Data Bank avec le code pdb suivant : 2GFI.

### 3- Propriétés enzymatiques

### 3.1 Effet du pH

L'effet du pH sur la phytase est déterminé, en mesurant l'activité enzymatique, en présence de différents tampons : pour les pH compris entre 2 et 3,5 (tampon glycine), pour les pH 3,5 à 7 (tampon acétate de sodium) et pour les pH 7 à 7,5 (tampon tris HCl) **(****Figure 1****).** La phytase est active entre des valeurs de pH de 2,5 à 6,5, avec un optimum entre pH 4 et 4,5. On peut noter un effet de la nature du tampon sur l'activité phytasique, à pH 3,5, le tampon acétate de sodium est inhibiteur par rapport au tampon glycine.

### 3.2 Effet de la température

La température optimale est déterminée sur la phytase native, en effectuant une mesure de l'activité phytasique à différentes températures : de 30 à 80°C (**Figure 2**).

La température optimale de la phytase est comprise entre 55 et 60°C. L'énergie d'activation calculée d'après la représentation d'Arrhenuis est de 38 kJ/mol.

### 3.3 Action des effecteurs

Parmi les différents cations testés, seul le Mn²⁺ provoque une forte inhibition de 72%. En présence des cations Co²⁺, Zn²⁺, Cu²⁺ et Mg²⁺, l'activité est inhibée de 58 à 22%. On peut noter que la présence de calcium n'est pas nécessaire à l'activité de cette enzyme **(tableau 3).**

**Tableau 3 : Influence des effecteurs sur l'activité phytasique de D. castellii.**

| Effecteurs | Concentration (mM) | % Activité |
|---|---|---|
| Témoin phytate | 9 | 100 |
| CaCl₂ | 5 | 90 |
| MnCl₂ | 5 | 28,8 |
| MgCl₂ | 5 | 68,7 |
| CuCl₂ | 5 | 66,6 |
| ZnCl₂ | 5 | 57 |
| FeCl₃ | 5 | 92 |
| CoCl₂ | 5 | 42,3 |
| CrCl₂ | 5 | 107,6 |
| HgCl₂ | 5 | 96,2 |
| PCMB | 3 | 94,5 |
| EDTA | 3 | 104,5 |
| Acide iodoacétique | 3 | 100 |
| 2-mercaptoéthanol | 3 | 107 |
| N-bromosuccinimide | 0,1 et 1 | 1 |
| N-bromosuccinimide + tryptophane | 1 + 3 | 100 |
| iode | 3 | 25,6 |
| iode + tryptophane | 1 + 3 | 88,1 |

Parmi les 6 inhibiteurs testés, seul le N-bromosuccinimide qui agit sur les groupements tryptophane, tyrosine et histidine inhibe totalement l'activité phytasique. L'ajout de tryptophane rétabli l'activité. L'iode, spécifique de groupements tyrosine, inhibe également fortement (75 %). Le tryptophane et la tyrosine semblent être fortement impliqués dans le site catalytique de l'enzyme.

Par contre, l'acide iodo acétique qui agit sur les groupements cystéine et histidine ne provoque aucune inhibition de l'activité.

L'absence d'effet du 2-mercaptoéthanol, de l'iodoacétate et du pCMB montre que les groupements -SH ne sont probablement pas impliqués dans le site catalytique.

### 3.4 Etude de la stabilité

### Température

Une étude de la dénaturation thermique à diverses températures montre que l'enzyme est stable pendant une heure à 60°C lorsqu'elle est dans de l'eau et une heure à 66°C lorsqu'elle est dans du tampon acétate 125 mM, pH 4 (Figure 3). Elle est dénaturée au dessus de 68°C, avec une perte d'activité de 70% après une heure à 70°C. L'énergie d'activation de la dénaturation calculée par la représentation d'Arrhenius est de 606 kJ/mol.

### pH

La phytase est totalement dénaturée après 21 jours de conservation à -20°C pour les pH inférieurs à 5. Par contre aucune dénaturation n'est observée après 67 jours de conservation à cette même température pour les pH compris entre 5 et 7.

### pH et température

L'activité phytasique est mesurée après incubation de l'enzyme à 2 températures (40 et 60°C) dans des tampons dont le pH est compris entre 2 et 8 **(****Figure 4****).** Pour les deux pH extrêmes (2 et 8) on constate une perte totale d'activité après 1 heure de contact. Par contre dans la gamme 3 à 7, de 80 à 100% d'activité sont conservés à 40°C. A 60°C, la phytase est plus fortement dénaturée aux pH 3 et 8. On peut noter qu'il y a un fort effet dû à la nature du tampon à pH 8 ; en présence de tampon acétate, la dénaturation est totale alors qu'en présence de tampon tris HCL, la dénaturation est de 50% seulement..

### Environnement- Force ionique

L'enzyme est pré incubée dans du tampon acétate 250 mM, pH 4 renfermant du chlorure de calcium 1 mM, à 20°C et 66,5°C pendant 60 minutes en présence de différents additifs. En fin de traitement, l'extrait est refroidi dans de l'eau à +4°C. L'activité est ensuite mesurée à 37°C pendant 20 minutes à pH 4, tampon 250 mM **(Tableau 4).**

Trois types d'éléments sont testés:
- sucres ou sucre alcool : sucrose, lactose, tréhalose, arabinose, glycérol,
- l'absence de calcium,
- la molarité du tampon.

L'enzyme est préincubé dans du tampon acétate 250 mM, calcium 1 mM, pH 4, à 20°C et 66,5°C pendant 60 minutes en présence de différents additifs. En fin de traitement, l'extrait est refroidi dans de l'eau à +4°C.

L'activité est ensuite mesurée à 37°C pendant 20 minutes à pH 4 tampon 250 mM, 1 mM de calcium (Cf. Matériels et Méthodes).

**Tableau 4 : Influence de l'environnement sur la thermostabilité de la phytase de Debaryomyces castellii CBS2923**

| **Eléments** | **Concentration** | **1 h à 20°C** | **1 h à 66,5°C** |
|---|---|---|---|
| **Contrôle** | | 99 | 47 |
| **CaCl₂** | 0 mM | 100 | 1 |
| **Sucrose** | 10% | 97 | 57 |
| **Lactose** | 10% | 95 | 47 |
| **Tréhalose** | 10% | 94 | 56 |
| **Arabinose** | 10% | 90 | 36 |
| **Glycérol** | 10% | 96 | 56 |
| **Tampon acétate** | 200 mM | 100 | 53 |
| **Tampon acétate** | 150 mM | 98 | 78 |
| **Tampon acétate** | 125 mM | 84 | 95 |
| **Tampon acétate** | 100 mM | 87 | 89 |
| **Tampon acétate** | 50 mM | 99 | 89 |

Deux éléments sont très importants, le calcium et la force ionique. Le calcium bien que n'ayant aucune influence sur l'activité enzymatique joue un rôle protecteur très important; la dénaturation est totale en absence de calcium. L'augmentation de la force ionique du tampon augmente la dénaturation par la température jusqu'à 50% à 250 mM.

### 4- Etudes cinétiques

### Etude de la spécificité

Les constantes cinétiques sont déterminées sur 2 substrats. L'affinité de la phytase est 4 fois plus forte en présence de phytate de calcium que de p nitro phenyl phosphate (pNPP) **(Tableau 5).**

**Tableau 5 : Spécificité de la phytase de D. castellii sur différents substrats**

| **Substrats** | **Km** **(mM)** | **Vm** **(µmol/min/mg)** |
|---|---|---|
| **pNPP** | 2,27 | 30,9 |
| **Phytate de sodium** | 0,532 | 35,8 |

Cette phytase possède un large spectre d'activité, avec une hydrolyse préférentielle du pNPP, du phosphoénol pyruvate, de l'ATP et de l'ADP **(Tableau 6A).** Elle se classe dans les phytases à large spectre telles que les phytases d'*A*. *fumigatus, E. nidulans.*

Elle dégrade également l'Ins (2) P₁, **(Tableau 6B**) qui est rarement hydrolysé par les phytases en raison de sa position axiale sur la molécule d'acide phytique. Son fonctionnement est inhibé par les phosphates avec un Ki de 1, 3 mM.

**Tableau 6 :** Comparaison de la spécificité de la phytase de *D. castellii* en présence de différents substrats.
**(A)** La concentration en substrats est de 4 mM. Les cinétiques de libération de phosphate sont réalisées dans du tampon acétate de sodium 250 mM, CaCl2 1 mM pH 4, à 37°C

| **Substrats** | **Concentration (mM)** | **% activité** |
|---|---|---|
| Acide phytique (témoin) | 9 | 100 |
| P-Nitrophénylphosphate | 4 | 135 |
| Fructose 6 phosphate | 4 | 35 |
| Glucose 6 phosphate | 4 | 82 |
| Adénosine 5' monophosphate | 4 | 50 |
| Adénosine 5' diphosphate | 4 | 120 |
| Adénosine 5' triphosphate | 4 | 133 |
| L-α-glycérophosphate | 4 | 78 |
| D (-) 3-phosphoglycéric acide | 4 | 95 |
| Phospho(enol)pyruvate | 4 | 130 |

**(B)** Hydrolyse de divers inositols phosphates par la phytase de *D. castellii.* Les cinétiques de libération de phosphates sont réalisées dans du tampon acétate de sodium 250 mM, CaCl₂ 1 mM, pH 4. Les mesures sont réalisées après 30 minutes d'hydrolyse à 37°C

| **Substrats** | **Concentration (mM)** | **% activité** |
|---|---|---|
| Acide phytique (témoin) | 9 | 91 |
| Ins(1)P₁ | 1 | 58 |
| Ins (2)P₁ | 1 | 84 |
| Ins (1,4) P₂ | 0,1 | 65 |
| Ins (1,4,5) P₃ | 0,1 | 56 |

### 5- Stéréospécificité

### 5.1 Séparation et identification des inositols phosphate par chromatographie (HPIC).

Les isomères de myo-inositol phosphate sont séparés sur une colonne analytique Omni Pac-100 en utilisant un gradient de 5% à 98% HCl (0,5 M) et H₂O/2-propanol (v/v). Les éluants sont mélangés dans un réacteur post-colonne avec une solution de 0,1% Fe(NO₃)₃, 9H₂O et HClO₄ 2% (Phillippy, B.Q. and Bland, J.M. (1988) Gradient ion chromatography of inositol phosphates. Anal. Biochem. 175, 162-166). Le débit total est à 1,2 mUmin. Ces conditions permettent de séparer les différents Ins Ps (Ins P₆ à Ins P₁) et d'identifier différents isomères d'Ins P₅ et d'Ins P₄.

Les différents isomères sont identifiés par comparaison des chromatogrammes obtenus avec (1) ceux décrits dans la littérature, obtenus dans des conditions identiques (Skoglund, E., Carlsson, N.G. and Sanberg, A.S. (1997) Determination of isomers of inositol mono- to hexaphophates in selected foods and intestinal contents using High-Performance Ion Chromatography. J. Agric. Food Chem. 45, 431-436), (2) avec les composés obtenus par hydrolyse chimique (Türk, M., Sandberg, A.S., Carlsson, N.G. and Andlid, T. (2000) Inositol hexaphosphate hydrolysis by baker's yeast. Capacity, kinetics, and degradation products. J. Agric. Food Chem. 48, 100-104), (3) avec les produits d'hydrolyse de l'acide phytique par les phytases d'*A. niger* et *P lycii* (Lassen, S.F., Bech, L., Fuglsang, C.C., Ohmann, A., Breinholt, J. and Stergaard, P.R. (2000), US Patent 6060298).

### 5.1.2 Hydrolyse chimique.

Après une hydrolyse chimique (HCl 6M, 100°C, 16 heures), le chromatogramme obtenu nous permet d'identifier 12 isomères parmi les Ins P₅ et Ins P₄. Les isomères d'Ins P₃, Ins P₂ et Ins P₁ ne sont pas séparés par cette méthode.

### 5.1.3 Identification des inositols phosphates formés au cours de l'hydrolyse de l'acide phytique par la phytase de D. castellii.

Les conditions d'hydrolyse sont décrites dans Matériels et Méthode. L'apparition des divers Ins Pₙ est suivie au cours du temps. Après 15 minutes d'hydrolyse, l'Ins P₆ à pratiquement disparu, 4 pics majoritaires sont détectés et correspondent à l'Ins (1,2,4,5,6) P₅, l'Ins (1,2,5,6) P₄, et des pics d'Ins P₃, Ins P₂ et Ins P₁ non identifiés par cette méthode (Figure 7). L'attribution des Ins P₅ et Ins P₄ est confirmé par ajout d'échantillons authentiques d'inositols. Après 120 minutes d'hydrolyse, les pics d'Ins P₃ et d'Ins P₁ sont majoritaires. Cela pourrait traduire une vitesse d'hydrolyse nettement plus faible de l'Ins P₃ par rapport aux Ins P₄, ₅ et ₆. Après 300 minutes d'hydrolyse la quantité de phosphate dosée correspond à 100% des phosphates potentiels de la molécule de phytate. L'enzyme libère la totalité des phosphates. La caractérisation des fractions d'Ins Pn séparées par chromatographie est réalisée par analyse RMN.

### 5.2 RMN (Figures 8-11)

### 5.2.1 Préparation des échantillons.

Les différents produits d'hydrolyse de l'Ins P₆ par la phytase de D. *castellii* sont séparés par chromatographie d'échange d'ions selon la méthode utilisée dans le paragraphe précédent. La quantité injectée correspond à 369,5 µg d'Ins P₆. Le réactif post-colonne est remplacé par de l'eau, 12 chromatographies sont réalisées.

Les fractions correspondant aux Ins P₅, Ins P₄, Ins P₃, Ins P₂, Ins P₁, sont collectées, rassemblées et lyophilisées. Les lyophilisats sont réhydratés avec 0,5 mL de D₂O et sont analysés par RMN à 17°C.

### 5.2.2 Analyse des InsP.

Comme cela a été détaillé dans Matériels et Méthodes les différentes fractions d'inositols sont caractérisées par l'analyse des spectres protons (1 D et 2D COSY et TOCSY) et de leurs spectres de corrélation 31P-1H (HMQC). S'il est possible de distinguer les stéréo-isomères, qui sont des images non miroir, il n'est pas possible de distinguer les énantiomères, qui donnent des images miroir et des spectres identiques.

De ce fait, la caractérisation RMN des produits d'hydrolyse ne permet pas de distinguer une 3-phytase d'une 1-phytase, et une 6-phytase d'une 4-phytase. Conventionnellement, d'après la littérature, lorsque les liaisons 3 ou 1 sont hydrolysées en premier on donne le nom de 3-phytase, de même pour les liaisons 6 ou 4 on donne le nom de 6-phytase.

### 5.2.2.1 Analyse de la fraction HPLC contenant les Ins P1.

Le spectre proton indique la présence d'un mélange de trois produits dont les systèmes de spin ont été identifiés à l'aide des expériences TOCSY et COSY. Ces trois produits sont : l'Ins (2) P1 (36%), l'Ins (1 ou 3) P1 (16%) et l'inositol (48%). Le spectre phosphore indique la présence de phosphore inorganique qui se caractérise par un signal fin. L'HMQC confirme que deux d'entre eux sont monophosphorylés et que le 3eme ne l'est pas, c'est l'inositol.

### 5.2.2.2 Analyse de la fraction HPLC contenant les Ins P2

Le spectre proton indique la présence d'un produit majoritaire (≈ 90%). Partant du signal correspondant au proton H2 (4,741 ppm), le COSY permet l'attribution du spectre. L'analyse des constantes de couplage indique que les positions 2 et 1 (ou 2 et 3) sont phosphorylées et que le produit majoritaire correspond à l'Ins (2,1 ou 2,3) P2. Le spectre découplé du 31 P et l'HMQC confirment cette attribution. Les produits minoritaires, au moins 2, n'ont pas été identifiés.

### 5.2.2.3 Analyse de la fraction HPLC contenant les l'Ins P3.

Le spectre proton indique la présence d'un produit pratiquement pur. L'analyse du spectre indique qu'il correspond à l'Ins (2,1,6 ou 2,3,4) P3. La phosphorylation de ces trois positions consécutives est confirmée par le spectre découplé du 31 P et par l'HMQC.

### 5.2.2.4 Analyse de la fraction HPLC contenant les Ins P4.

Le spectre proton indique la présence d'un produit majoritaire (≈ 90%) qui se révèle être l'Ins (2,1,6,5 ou 2,3,4,5) P4. Les positions des groupements phosphates déduites de l'analyse des constantes de couplages (3JHCOP = 8-10 Hz) sont confirmées par les expériences de découplage 31 P et par l'expérience HMQC. Les produits minoritaires n'ont pas été identifiés.

### 5.2.2.5 Caractérisation de l'Ins P5

Compte tenu que l'InsP5 est le premier produit formé et qu'il est aussitôt dégradé en InsP4 il ne nous a pas été possible d'en isoler suffisamment par HPLC pour pouvoir le caractériser par RMN.

Afin de contourner cette difficulté, l'Ins P5 a été caractérisé *in situ* au cours d'une cinétique d'hydrolyse réalisée à 17°C avec une faible quantité de phytase (voir ci-dessous). Dans ces conditions, les spectres protons (spectre de différence 1D, TOCSY et COSY) de l'Ins P5 ont pu être obtenus. L'ensemble de ces spectres permet de caractériser l'Ins (1,2,4,5,6) P5 indiquant que la position 3 (ou 1) est la première position à être déphosphorylée. Le spectre 31 P de l'Ins P5 n'a pas été enregistré (Figure 8).

### 5.2.3 Cinétique d'hydrolyse de l'InsP6

Des études préliminaires ont permis d'une part de montrer que l'enzyme était insensible à la présence de deutérium et d'autre part d'ajuster la concentration en enzyme pour avoir une cinétique ni trop rapide ni trop lente par rapport aux contraintes techniques d'observation.

En fonction de la température (ici 17°C) et de la concentration en enzyme, une cinétique peut être lente (1 µl d'enzyme) et l'apparition des premiers intermédiaires aisément observée. C'est dans ces conditions que les caractéristiques spectrales de l'InsP5 ont été obtenues et que l'on voit clairement apparaître successivement l'Ins P5, l'Ins P4 et l'Ins P3.

En présence de 5 µl d'enzyme, la formation des Ins P2, Ins P1 et d'inositol est observée. Dans la phase intermédiaire, la complexité des spectres obtenus rend compte du mélange des Ins Pn. Après 10 heures, l'hydrolyse n'est pas totale. En présence de 20 µl d'enzyme, une hydrolyse totale a été obtenue en environ 4 heures. Ces résultats cinétiques confirment l'hydrolyse des 6 liaisons phosphate précédemment observée. Ils montrent clairement que l'hydrolyse des 2 dernières liaisons, sont nettement plus lente que les autres. L'effet inhibiteur du phosphate qui s'accumule dans le milieu en serait en partie responsable **(****Figures 9****,** **10****,** **11****).**

Dans cette étude, nous avons décrit les voies d'hydrolyse de l'acide phytique par la phytase de *D. castellii.* L'analyse par chromatographie et par RMN homo et hétéronucléaire a permis de déterminer sans ambiguïté la structure des principaux et différents Ins Ps formés: Ins P5, Ins P4, Ins P3, Ins P2, Ins P1. La séquence de déphosphorylation est présentée sur la Figure 5. Elle peut être résumée sous la forme 3/4/5/6/1,2. Bien que les isomères de position 1 et 3 et 4 et 6 ne soient pas discernables par RMN, nous avons cependant grâce aux analyses en HPIC pu déterminer que les groupements phosphates position 3 puis 4 sont les deux premiers à être hydrolysés. La cinétique suivie par RMN confirme cette observation. Les hydrolyses suivantes se situent sur le phosphate adjacent au groupe hydroxyle. L'hydrolyse des 2 dernières liaisons phosphate Ins (1,2) P2 se fait simultanément. Cependant, la vitesse d'hydrolyse de la position 2 semble deux fois plus faible que celle de la position 1 (ou 3). Enfin, quelle que soit la méthode utilisée (enzymatique, HPLC ou RMN), l'obtention quantitative d'inositol en fin d'hydrolyse montre que les six liaisons phosphate sont hydrolysées par la phytase de la levure *D. castellii.* Cette phytase peut être classée comme une 3-phytase (EC 3.1.3.8) semblablement à de nombreuses phytases de microorganismes.

### Exemple 2 : Clonage, surexpression et caractérisation biochimique de la phytase recombinante

### MATERIELS ET METHODES

### Organismes, vecteurs et séquences oligonucléotidiques

Les souches, plasmides et amorces utilisés dans cette étude sont décrits dans le **tableau 7.**

**Tableau 7 : Souches, plasmides et oligonucleotides utilisés pour cette étude**

| **Souches, plasmides ou amorces** | **Description ou caractéristiques** | **Référence ou source** |
|---|---|---|
| Souches | | |
| *D. castellii* CBS 2923 | Producteur de phytase | CBS, Delft (NL) |
| *E. coli* XL1-Blue MRF | Δ(*mcr*A)*183* Δ(*mcr*CB-*hsd*SMR-*mrr*)173 *end*A1 *sup*E44 *thi*-1 *rec*A1 *lac* [F'*pro*AB *lac* I^{q}ZΔM15 Tn*10*(Tet^{r})] | Stratagene |
| *P. pastoris* X33 | | Invitrogen |
| | | |
| Plasmides | | |
| pGEM-T | Contient le gène conférant la résistance à l'ampicilline | Promega |
| pPICZαB | Promoteur AOX1 inductible; séquence signal du facteur α de *S*. *cerevisiae*; gène *Sh ble* conférant la résistance à la zéocine; intégratif | Invitrogen |
| pGAPZαB | Promoteur GAP constitutif; séquence signal du facteur α de *S*. *cerevisiae*; gène *Sh ble* conférant la résistance à la zéocine; intégratif | Invitrogen |
| | | |
| Amorces | | |
| phyt-Nter-for | 5'-TCIAA(A/G)TT(A/G)AT(T/C/A)AA(T/C)AA(T/C)GG-3' Peptide correspondant : SKLINNG | |
| phyt-pep1-rev | 5'-GG(A/T/C/G)AC(A/G)AA(A/G)TA(C/T)TC(A/G)TA(A/G)TC-3' Peptide correspondant : PVFYEYD | |
| AP1 | 5'-GTA ATA CGA CTC ACT ATA GGG C-3' | BD Biosciences |
| AP2 | 5'-ACT ATA GGG CAC GCG TGG T-3' | BD Biosciences |
| 5phyt-spe-1 | 5'-TATGGAGCAGCTCCTCCTAAGAATCTG-3' | |
| 5phyt-spe-2 | 5'-ATGATGTTATATTGCTCGACGGACGCTTG-3' | |
| 3phyt-spe-1 | 5'-TATGGAGCAGCTCCTCCTAAGAATCTG-3' | |
| 3phyt-spe-2 | 5'-CTGGCTCCGGAAAGAAATATAAGGCTGTA-3' | |
| 3bisphyt-spe-1 | 5'-GAAGTGTAGCTCTGGTCCTGGTTTCTCATG-3' | |
| 3bisphyt-spe-2 | 5'-ATGTTGCTGAAAGAGTTGCAGGTACCAACT-3' | |
| phytDc-PstI-for | 5'-GCACTGCAGTCTCAGTCTCAAAGTTAATTAAC-3' | |
| phytDc-XbaI-rev | 5'-AGTTCTAGATTAACTGTTGATAAGGGAAGCGGT-3' | |

La levure *Debaryomyces castellii* CBS 2923 est cultivée en Erlenmeyer remplis au 1/10^{ème} de leur volume en conditions aérobies et à 28°C. Lors de l'extraction de l'ADN génomique, la souche est cultivée en présence de 500 mL de milieu YPD ou de 20 mL de milieu MSA.

La souche *E. coli* XL1-Blue MRF', utilisée lors de l'amplification de l'ADN, est cultivée en Erlenmeyer remplis au 1/10^{ème} de leur volume en conditions aérobies et à 37°C, en présence de milieu Luria-Bertani (Sambrook; J., Fritsch, E. F. and Maniatis, T. (1989) Molecular cloning : a laboratory manual. 2nd edn. Cold Spring Harbor Laboratory Press, Cold Spring Harbor, New York, USA) additionné d'ampicilline (100 mg/L) ou de milieu Luria-Bertani "low salt" (NaCl 5 g/L) dont le pH est ajusté à 7,5 avec de la soude et additionné de zéocine (25 mg/L). Lors de culture en milieu solide, les milieux précédents sont additionnés de 15 g/L de Bacto agar.

La souche de levure *Pichia pastoris* X33 est utilisée pour l'expression hétérologue du gène codant la phytase. Les transformants sont sélectionnés, en conditions aérobies, à 28°C, sur milieu gélosé YPDS, additionné de zéocine (100 mg/L).

Le vecteur pGEM-T (Promega) est utilisé pour le clonage dans *E. coli* de fragments amplifiés par réaction de polymérisation en chaîne (RPC) et pour le séquençage de l'ADN. Le marqueur de sélection de ce plasmide est le gène de résistance à l'ampicilline. Deux vecteurs d'expression, pPICZaB et pGAPZaB sont utilisés (**tableau 7**). Le vecteur pPICZaB contient le promoteur du gène de l'alcool (méthanol) oxydase (AOX1) qui permet d'obtenir de forts taux d'expression du gène d'intérêt dans *P. pastoris* et l'expression de la protéine recombinante est inductible par le méthanol (Cereghino, J. L. and Cregg, J. M. (2000) Heterologous protein expression in the methylotrophic yeast Pichia pastoris. Fems Microbiology Reviews 24, 45-66). Le vecteur pGAPZαB contient le promoteur du gène de la glycéraldéhyde-3-phosphate déshydrogénase (GAP) qui permet une expression constitutive et à fort taux de la protéine recombinante dans *P. pastoris* (Waterham, H. R., Digan, M. E., Koutz, P. J., Lair, S. V. and Cregg, J. M. (1997) Isolation of the Pichia pastoris glyceraldehyde-3-phosphate dehydrogenase gene and regulation and use of its promoter. Gene 186, 37-44). Ces deux vecteurs possèdent également les séquences nécessaires pour la réplication dans les bactéries. La sélection des clones transformés par ces vecteurs est basée sur un marqueur de sélection, la zéocine, et cette sélection est applicable à la fois chez *P. pastoris* et *E. coli.* De plus, les protéines recombinantes sont exprimées en fusion avec la séquence signal du facteur α de *S. cerevisiae.*

### Milieux

*D. castellii* CBS 2923 est cultivée en présence de milieu YPD (extrait de levures 10 g/L, peptone 20 g/L, glucose 20 g/L) ou de milieu MSA-B (cf. exemple 1 matériels et méthodes) tamponné à pH 5.4 par du tampon phtalate 100 mM. Après transformation, les transformants *P. pastoris* X33 sont sélectionnés sur milieu YPDS solide (extrait de levures 10 g/L, peptone 20 g/L, glucose 20 g/L, sorbitol 1 M, Bacto agar 20 g/L). Lors de la sélection des clones producteurs en milieu gélosé, les transformants *P. pastoris* sont cultivés sur un milieu solide synthétique contenant du glucose ou du méthanol à 0.5 %, du Bacto agar 20 g/L, des sels FM21 100 mL/L (FM21 10X : CaSO₄2H₂O 1.5 g/L, K₂SO₄ 23.8 g/L, MgSO₄7H₂O 19.5 g/L, KOH 6.5 g/L, H₃PO₄ 85 % 3.5 % v/v), des oligoéléments PTM1 10 mL/L (PTM1 100X : ZnCl₂ 2 g/L, Fe(SO₄)7H₂O 6.5 g/L, CuSO₄5H₂O 0.6 g/L, MnSO₄H₂O 0.3 g/L, KI 10 mg/L, H₃BO₄ 2 mg/L, Na₂MoO₄ 20 mg/L, H₂SO₄ 96 % 0.2 %), du (NH₄)₂SO₄ 4 g/L, du H2PO₄NH4 12 g/L et de la D-biotine 80 µg/L, tamponné à pH 5.4 par du tampon tartrate-phosphate 200 mM. Lors de la sélection des clones producteurs en milieu liquide, les transformants *P. pastoris* sont cultivés en présence d'un milieu synthétique contenant une source de carbone (glucose 10 g/L, glycérol 20 g/L ou méthanol 3.9 g/L), des sels FM21 100 mL/L, des oligoéléments PTM1 10 mL/L, du (NH₄)₂SO₄ 4 g/L, du H₂PO₄NH₄ 12 g/L et de la D-biotine 80 µg/L, tamponné à pH 5.4 par du tampon tartrate-phosphate 200 mM. Lors des cultures en bioréacteur, les précultures sont réalisées en présence de milieu YMPG (extrait de levures 3 g/L, extrait de malt 3 g/L, peptone 5 g/L, glucose 10 g/L) puis en présence de milieu synthétique : glycérol 40 g/L, sels FM21 100 mL/L, oligoéléments PTM1 10 mL/L, (NH₄)₂SO₄ 4 g/L, H₂PO₄NH₄ 12 g/L et D-biotine 80 µg/L, tamponné à pH 5.4 par du tampon tartrate-phosphate 200 mM (Klein et al., 1998). Les cultures en mode batch sont réalisées en présence de milieu synthétique contenant du glycérol 40 g/L ou du glucose 20 g/L, des sels FM21 100 mL/L, des oligoéléments PTM1 10 mL/L et de la D-biotine 80 µg/L. Lorsqu'une seconde culture batch est réalisée, une solution de glycérol est ajoutée dans le fermenteur de manière à avoir une concentration finale en glycérol de 40 g/L. Cet ajout est réalisé après consommation totale du glycérol initialement présent, ceci étant visualisable par une augmentation brusque du taux d'oxygène dissout. Les cultures en mode fed-batch sont alimentées par du milieu synthétique contenant du glycérol à 400 g/L, du glucose à 400 g/L ou du méthanol à 780 g/L, des oligoéléments PTM1 50 mL/L et de la D-biotine à 1 mg/L. La culture en mode continu est alimentée par du milieu synthétique : méthanol 66.67 g/L, sels FM21 50 mL/L, oligoéléments PTM1 25 mL/L et D-biotine 500 µg/L.

### Extraction d'ADN génomique

### Extraction rapide d'ADN génomique (ADNg)

Les cellules provenant d'une culture de *D. castellii* CBS 2923 en présence de milieu MSA sont récoltées après 9 h de culture, centrifugées (3 500 *x g,* 20 min, +4°C) et lavées dans 10 mL d'eau stérile. Les cellules sont ensuite remises en suspension dans 200 µL de tampon de lyse (Tris-HCl 10 mM, pH 8, EDTA 1 mM, NaCl 100 mM, triton X-100 2 % v/v, SDS 1 % p/v), 200 µL de billes de verre (diamètre 0.45-0.5 mm) et 200 µL de mélange phénol/chloroforme/alcool isoamylique (25/24/1; v/v/v). La lyse des cellules est effectuée par agitation vigoureuse pendant 3 min puis 200 µL de tampon TE (Tris-HCl 10 mM, EDTA 1 mM, pH 8) sont ajoutés. La solution est centrifugée (8 000 *x g*, 5 min, +4°C). Les protéines sont extraites de la phase aqueuse avec un volume de mélange chloroforme/alcool isoamylique (24/1; v/v), centrifugation (8 000 *x g,* 2 min, +4°C). L'ADN génomique est précipité avec 1 mL d'éthanol absolu (-20°C), centrifugé (12 000 *x g,* 5 min, +4°C) et remis en solution dans 370 µL de tampon TE. L'ARN est éliminé par incubation 15 min à +37°C en présence de 75 µg/mL de RNase A. L'ADN est ensuite précipité avec de l'éthanol absolu (-20°C), remis en suspension dans 50 µL de tampon TE et conservé à -20°C.

### Préparation d'ADN liquide de haut poids moléculaire de levure

Les cellules provenant d'une culture de *D. castellii* CBS 2923 en présence de 500 mL de milieu YPD sont récoltées pendant la phase exponentielle, centrifugées (6 000 *x g,* 15 min, +4°C) et lavées dans 40 mL d'eau stérile. Le culot est remis en solution dans 3.5 mL de solution SCE (sorbitol 1 M, citrate de sodium 0.1 M, EDTA 60 mM) contenant 40 µL de dithiothréitol 2 M et 5 mg (5 000 unités) de zymolase 100-T (ICN Biomedical, 32093) et incubé pendant 1 h à +37°C. 7 mL de la solution de lyse (Tris-HCl 0.5 M, pH 6.5, sarcosyl sodium 3.2 % p/v, EDTA 0.2 M, protéinase K 100 µg/mL (Roche Diagnostics, Meylan, France) sont ensuite ajoutés et la suspension est incubée pendant 15 min à +65°C puis rapidement refroidie à température ambiante.

Un gradient de saccharose est préparé en versant successivement dans un tube d'ultracentrifugation 11 mL de saccharose 20 % p/v, 11 mL de saccharose 15 % p/v puis 3 mL de saccharose 50 % p/v. La suspension cellulaire lysée est délicatement déposée sur le gradient puis ultracentrifugée pendant 3 h à 26 000 rpm à +20°C (ultracentrifugeuse Centrikon T-1075, Kontron Instrument). L'ADN, qui forme un voile dans le gradient, est récupéré à la pipette, précipité à l'éthanol et remis en suspension dans 100 µL de tampon TE (Tris-HCl 10 mM, EDTA 1 mM, pH 8). L'ARN est éliminé par incubation pendant 3 h à +37°C en présence de 50 µg/mL de RNase A. L'ADNg est à nouveau précipité à l'éthanol puis remis en suspension dans 200 µL de tampon TE et conservé à -20°C.

### Clonage du gène de la phytase

### Amplification d'un premier fragment du gène

Une réaction de polymérisation en chaîne (RPC) est réalisée en utilisant comme matrice l'ADNg de *D. castellii* CBS 2923, obtenu par la méthode d'extraction rapide d'ADN génomique. La paire d'amorces phyt-Nter-for/phyt-pep1-rev est dessinée à partir de séquences de la protéine en limitant au maximum le taux de dégénérescence (**tableau 7**). L'ADN est amplifié grâce à la polymérase Taq (Promega) à l'aide du thermocycleur Minicycler (MJ Research). Après une première dénaturation de l'ADNg pendant 3 min à +94°C, l'amplification est effectuée en 30 cycles selon le programme de température suivant : 1 min de dénaturation à +94°C, 1 min d'hybridation à +45°C puis 1 min de polymérisation à +72°C. Ces cycles sont suivis d'un pallier de 10 min à +72°C.

### Clonage de la séquence complète du gène de la phytase

La séquence complète du gène est obtenue en utilisant la technique de "marche sur le génome" (Universal Genome Walker Kit, BD Biosciences) conformément aux instructions du fournisseur. L'ADNg de *D. castellii* CBS 2923 (2.5 µg), extrait par la méthode "préparation d'ADN liquide de haut poids moléculaire de levure", est digéré pendant 16 h à +37°C par quatre enzymes de restriction séparément (50 unités de *Dra*I, *Eco*RV, *Pvu*II ou *Stu*I). Chaque fragment possède ainsi des extrémités franches. Les fragments sont purifiés par extraction au phénol/chloroforme et précipités à l'éthanol. Les fragments sont ensuite liés aux adaptateurs fournis avec le kit de "marche sur le génome" pendant 16 h à +16°C. La ligation est arrêtée par chauffage à +70°C pendant 5 min puis addition de 9 volumes de tampon TE. Quatre banques sont ainsi construites et nommées "banque *Dra*I, *Eco*RV, *Pvu*II et *Stu*I". Un premier cycle de marche sur le génome est réalisé en utilisant chaque banque comme matrice et un couple d'amorces comportant l'amorce AP1 spécifique de l'adaptateur et les amorces 5phyt-spe-1/3phyt-spe-1 spécifiques du gène (**tableau 7**). L'efficacité de ce premier cycle de RPC est vérifiée sur gel d'électrophorèse d'agarose LE 1.5%. Le mélange, issu de la première RPC, est dilué 50 fois et sert de matrice pour le deuxième cycle de RPC. Ce deuxième cycle est réalisé en utilisant l'amorce AP2 spécifique du deuxième adaptateur et les amorces 5phyt-spe-2/3phyt-spe-2 spécifiques du gène (**tableau 7**). Les fragments ainsi amplifiés sont séparés sur gel d'électrophorèse d'agarose 1.2 % à bas point de fusion, purifiés à l'aide d'un kit "GeneClean" et clonés dans le vecteur pGEM-T afin d'être séquencés. Un deuxième cycle de marche sur le génome a été nécessaire pour obtenir la séquence complète du gène. Pour chaque banque, deux cycles d'amplification par RPC sont réalisés comme précédemment en utilisant les amorces AP1/3bisphyt-spe-1 pour le premier cycle et AP2/3bisphyt-spe-2 pour le deuxième cycle (**tableau 7**).

### Construction des plasmides d'expression

De manière à cloner le gène *phytDc* dans *P. pastoris* à l'aide des vecteurs d'expression pPICZαB et pGAPZαB, la séquence complète du gène est amplifiée par RPC. Cette RPC est réalisée en utilisant comme matrice l'ADNg de *D. castellii* CBS 2923, extrait par la méthode d'extraction rapide d'ADN génomique, à l'aide de la polymérase *Pfu* Turbo (Stratagene). Lors de cette amplification, la paire d'amorces utilisée, phytDc-Pstl-for et phytDc-Xbal-rev (**tableau 7**), a permis la création de sites de restriction *Pst*I et *Xba*I respectivement en 5' et en 3' du gène. Après une première dénaturation de l'ADNg pendant 2 min à +95°C, l'amplification est effectuée en 25 cycles selon le programme de température suivant : 30 s de dénaturation à +95°C, 30 s d'hybridation à +55°C puis 1.5 min de polymérisation à +72°C. Ces cycles sont suivis d'un pallier de 10 min à +72°C. L'absence de mutation est vérifiée par séquençage.

### Transformation

Les cellules d'*E. coli* XL1-blue MRF' sont transformées par choc thermique comme décrit par Sambrook et al. (Sambrook, J., Fritsch, E. F. and Maniatis, T. (1989) Molecular cloning : a laboratory manual. 2nd edn. Cold Spring Harbor Laboratory Press, Cold Spring Harbor, New York, USA). Les cellules de *P. pastoris* X33 sont transformées par électroporation (Manuel d'instruction Invitrogen pPICZaA, B et C, version E ou manuel d'instruction Invitrogen pGAPZaA, B et C, version F, Invitrogen Ltd, UK). Les transformations sont réalisées à l'aide d'un appareil Gene Pulseur (Biorad) réglé à un voltage de 1.5 kV et une capacitance de 25µF et un Pulse Controller (Biorad) réglé à 200 ohms.

### Synthèse et analyse des séquences

Les oligonucléotides utilisés comme amorces de RPC ont été synthétisés par la société MWG-Biotech (Allemagne). Le microséquençage de la séquence N-terminale et de peptides internes de la phytase a été effectué à l'aide d'un microséquenceur de protéines (Beckman/porton LF 3000) en utilisant les réactifs et méthodes recommandés par le fabriquant. Le microséquenceur était connecté directement à un système chromatographique de type RP-HPLC (Beckman 125S) avec un détecteur UV à 268 nm (Beckman 166). Les données sont traitées avec le logiciel Gold V8.20. La recherche d'alignements locaux entre des séquences nucléotidiques ou protéiques et des banques de données généralistes est effectuée avec les logiciels BLAST2 (Basic Local Alignment Search Tool) et FASTA. La comparaison entre deux séquences est effectuée avec les programmes LALIGN et LFASTA, nucléique ou protéique, du package FASTA. Ces programmes sont disponibles sur le serveur Infobiogen (http://www.infobiogen.fr). La prédiction de peptide signal et le calcul des pHi théoriques à partir de séquences protéiques sont effectués avec le logiciel Antheprot 2000 V5.2 release 1.1.6. Les sites de glycosylation sont prédits à l'aide des serveurs NetNGlyc 1.0, DictyOGlyc 1.1 et NetOGlyc 2.0, Center for Biological Sequence Analysis, Technical University of Denmark.

### Sélection des clones producteurs en milieu liquide

Les transformants *P. pastoris* pGAPphyt sont cultivés à +28°C en présence de 5 mL de milieu synthétique contenant 10 g/L de glucose. Après 48 h, les cultures sont centrifugées pendant 5 min à 1676 *x g*. Les surnageants sont conservés à +4°C. Les transformants *P. pastoris* pPICphyt sont cultivés à +28°C en présence de 5 mL du même milieu contenant 20 g/L de glycérol au lieu du glucose. Après 48 h, les cultures sont centrifugées pendant 5 min à 1676 *x g*. La production de recPhyt est induite en ajoutant 2.5 mL du même milieu contenant 3.9 g/L de méthanol au lieu du glucose dans les tubes contenant les cellules. Après 24 h d'induction, les cultures sont centrifugées pendant 5 min à 1676 *x g*. 2.5 mL du même milieu contenant 3.9 g/L de méthanol au lieu du glucose sont ajoutés dans les tubes contenant les cellules. Après 24 h d'induction, les cultures sont centrifugées pendant 5 min à 1676 *x g*. Les surnageants sont conservés à +4°C. Ces cultures sont réalisées dans des tubes stériles de 13 mL.

### Production de phytase recombinante

### Précultures en Erlenmeyer

Les précultures sont réalisées en présence de milieu YMPG en Erlenmeyer remplis au 1/10^{ème} de leur volume. Elles sont incubées à +28°C sous agitation (80 oscillations par min, amplitude 7 cm) pendant 24 h. Les précultures sont ensuite réalisées en présence de milieu synthétique pendant 24 h.

### Cultures en bioréacteur

Les cultures en mode batch, fed-batch et continu sont réalisées dans des fermenteurs Applikon de 1 ou 3 L (The Netherlands). Le pH est ajusté à 4 par des ajouts automatiques d'NH₃ 16 % (v/v) ou d' H₂SO₄ 2 N et la température est maintenue à +28°C. Afin d'éviter la formation de mousse, de l'antimousse Biospumex 153K (Cognis Dusseldorf, Allemagne) est ajouté au cours de la culture : antimousse 5 % (v/v) pour les cultures en mode fed-batch ou 1 % (v/v) pour les cultures en mode continu. La pression partielle en oxygène dissous est mesurée à l'aide d'une sonde Applisens (The Netherlands) et maintenue à une valeur supérieure à 30% par régulation de l'agitation et de l'aération. L'acquisition des données et le contrôle des cultures sont effectués grâce au logiciel BioExpert d'Applikon.

### Conditions de culture

Voir exemple 1 culture en fermenteur.

### Détermination de la concentration en biomasse

Les concentrations cellulaires sont mesurées par densité optique (DO) à l'aide d'un spectrophotomètre (DU530, Beckman Instruments Inc., Fullerton, CA, USA) à 600 nm.

Une unité de DO correspond à 0.462 g de matière sèche/L (g MS/L) sur glycérol, 0.442 g MS/L sur méthanol et 0.491 g MS/L sur glucose.

### Purification de la phytase recombinante

Voir exemple 1 , matériels et méthodes 3.

### Electrophorèse SDS-PAGE

Voir exemple 1, matériels et méthodes 4.

### Détermination de la concentration en protéines

Voir exemple 1, matériels et méthodes 7.2.

### Techniques enzymatiques

### Détection de l'activité phytasique en milieu gélosé

L'activité phytasique est détectée en utilisant la méthode de Kim et al. (Kim, Y. O., Kim, H. K., Bae, K. S., Yu, J. H. and Oh, T. K. (1998) Purification and properties of a thermostable phytase from Bacillus sp. DS11. Enzyme Microb. Technol. 22, 2-7) modifiée pour la révélation spécifique des phosphatases en gel de polyacrylamide. Les transformants *P. pastoris* sont cultivés en conditions aérobies à +30°C sur milieu synthétique solide contenant 0.5 % de glucose ou de méthanol selon le vecteur utilisé. Ces cultures sont incubées pendant 3 jours. Dans le cas des clones *P. pastoris* transformés avec le vecteur pPICZaB modifié, 50 µL de méthanol sont ajoutés quotidiennement dans le couvercle des boîtes de Pétri à partir du troisième jour. La solution de révélation (Bacto agar 10 g/L, α-naphtylphosphate 2 g/L, FastGarnetGBC 1 g/L, phytate de sodium 0.92 g/L, tamponné à pH 5.5 par du tampon acétate de sodium 0.25 M) est préparée juste avant utilisation, refroidie à 45°C et versée en fine couche sur les colonies. La coloration (brun, rouge foncé) des clones producteurs de phytase apparaît immédiatement.

### Détermination de l'activité phytasique par hydrolyse du phytate

Voir exemple 1, matériels et méthodes 7.3.

### Détermination de l'activité phytasique par hydrolyse du p-nitrophénylphosphate (p-NPP)

La détermination de l'activité est effectuée par réalisation de cinétiques enzymatiques. Le milieu réactionnel (2 volumes au total), tamponné à pH 5,5 (acétate de sodium 125 mM) contient 6 mM de p-nitrophénylphosphate et l'extrait enzymatique dilué ou non (1 volume). Après différentes durées d'incubation à température ambiante, la réaction est arrêtée par ajout d'hydroxyde de sodium 0,3 N (1 volume) pour basifier le milieu et révéler la coloration. L'absorbance est mesurée à 450 nm à l'aide d'un spectrophotomètre lecteur de microplaque Sanofi Pasteur PR 2100. L'unité enzymatique (U) est définie comme la quantité d'enzyme libérant une micromole de p-nitrophénol par min à partir de la solution de p-nitrophénylphosphate dans des conditions définies: température ambiante, pH 5,5. Une droite d'étalonnage a préalablement été établie avec du p-nitrophénylphosphate (0.012-0.12 µmol).

### Etudes cinétiques

Voir exemple 1, 7.5 et 7.6.

### RESULTATS

### Caractérisation du gène de la phytase

### Clonage du gène de la phytase de D. castellii CBS 2923

Un peptide interne et le peptide N-terminal de la phytase pure de D. *castellii* ont été séquencés. A partir de ces séquences peptidiques, des amorces de RPC sont dessinées, en limitant au maximum le taux de dégénérescence (**tableau 7**). Une RPC est réalisée en utilisant comme matrice l'ADNg de *D. castellii* CBS 2923. Un fragment d'environ 300 paires de bases (pb) a ainsi pu être amplifié. De manière à cloner la séquence complète du gène de la phytase de *D. castellii* CBS 2923, nous avons choisi d'utiliser la technique de "marche sur le génome". A partir de la séquence du fragment de 300 pb obtenue précédemment, des amorces correspondant aux extrémités du fragment et permettant d'amplifier les séquences adjacentes de ce fragment sont synthétisées (**tableau 7**). Une RPC est réalisée en utilisant les banques d'ADNg de *D. castellii* CBS 2923 comme matrices. Le premier cycle a permis d'amplifier un fragment d'environ 1600 pb en amont et d'environ 1100 pb en aval de la séquence de 300 pb à partir des banques *Eco*RV et *Pvu*II respectivement. Ainsi, nous avons pu reconstituer un fragment d'environ 3000 pb comportant une phase ouverte de lecture (ORF) de 1325 pb tronquée en 3'. Un deuxième cycle de RPC a été réalisé en utilisant de nouvelles amorces dessinées à partir de l'extrémité 3' du fragment obtenu lors de la première RPC. Cette RPC a permis d'amplifier un fragment d'environ 100 pb à partir de la banque *Eco*RV. Nous avons pu ainsi reconstituer la phase ouverte de lecture de 1386 pb. La séquence protéique déduite de cette ORF contient les deux peptides séquencés précédemment ce qui permet de confirmer que ce gène correspond au gène codant la phytase de *D. castellii* CBS 2923 (voir SEQ ID No.1). Afin de vérifier la séquence, des amorces correspondant aux extrémités du gène ont été synthétisées (**tableau 7**). Le gène complet a été amplifié par RPC et le séquençage a confirmé les résultats obtenus précédemment.

### Analyse des séquences nucléotidique et protéique

Grâce aux expériences de "marche sur le génome", un fragment de 2990 pb a pu être reconstitué. Ce fragment contient une phase ouverte de lecture de 1386 pb appelée « séquence *phytDc* ». La séquence protéique de 461 acides aminés correspondante contient plusieurs motifs (SEQ ID No. 2). Un motif RHGERYP correspond à la séquence consensus RHGXRXP présente dans le site actif de nombreuses phosphatases acides de haut poids moléculaire. La séquence possède également le motif HD dans la partie C-terminale, motif présent dans de nombreuses séquences de phytases. La séquence N-terminale débute dès le deuxième acide aminé de la protéine déduite ce qui semble indiquer que cette protéine, bien qu'excrétée, ne possède pas de peptide signal. Cette observation est appuyée par l'obtention d'un résultat négatif lors de la recherche d'un site potentiel de clivage de peptide signal à l'aide du logiciel Antheprot. La protéine déduite a une masse moléculaire estimée de 51.2 kDa, proche de la masse obtenue expérimentalement (53/55 kDa) par spectrométrie de masse à partir de la protéine exprimée dans *D. castellii* CBS 2923, purifiée et déglycosylée par l'endoglycosydase H. La différence de masse peut s'expliquer par la présence de résidus N-acétyl glucosamine ou de motifs de O-glycosylation qui ne sont pas éliminés par l'endoglycosylase H. L'analyse de la séquence protéique montre la présence de 9 sites potentiels de N-glycosylation et 4 sites potentiels de O-glycosylation. Le pHi de cette protéine est estimé à 4.3. La recherche d'homologies avec des banques de données montre que la phytase de *D. castellii* CBS 2923 possède de 21 à 36 % d'homologies avec diverses phytases d'origine levurienne ou fongique. Les tailles des séquences de ces phytases sont très proches (440-480 acides aminés) mais la phytase de *D. castellii* CBS 2923 ne s'aligne pas parfaitement sur toute la longueur de sa séquence. La séquence *phytDc* partage, sur toute sa longueur, 69.2 % d'homologie avec la séquence de la phytase de *S. occidentalis.* La séquence du gène codant la phytase de S. *occidentallis* ayant été obtenue à partir de l'ADN complémentaire, cet alignement nous permet de penser que le gène codant la phytase de *D. castellii* ne possède pas d'introns. Par ailleurs, cette séquence possède des homologies avec des phosphatases.

### Sélection des clones de P. pastoris hyperproducteurs de phytase recombinante

Le gène *phytDc* a été amplifié par RPC et inséré dans les vecteurs d'expression pPICZaB et pGAPZaB. Les transformants ont été sélectionnés sur milieu YPDS contenant de la zéocine. Le taux de transformation est de 10² transformants par µg d'ADN. Les clones obtenus avec le vecteur pPICZαB sont appelés "clones PIC" et les clones obtenus avec le vecteur pGAPZaB sont appelés "clones GAP" Pour chaque transformation, 100 clones ont été prélevés au hasard et leur production de phytase recombinante (recPhyt) a été évaluée en milieu gélosé. Après révélation, les clones les plus producteurs présentent un halo brun autour des colonies. 96 % des clones PIC et 66 % des clones GAP sont producteurs. Parmi les clones les plus producteurs, 10 clones GAP et 10 clones PIC ont été testés sur milieu synthétique en Erlenmeyer. Pour chaque clone, le rapport entre la biomasse (évaluée par la mesure de la D.O.) et l'activité phytasique dans le surnageant de culture (mesurée par l'hydrolyse du p-NPP) a été déterminé. Le rapport varie de 0 à 0.48 parmi les clones PIC et de 1.28 à 6.07 pour les clones GAP. Dans ces conditions de culture, les clones GAP sont les meilleurs producteurs. Selon Waterham et al. (Waterham, H. R., Digan, M. E., Koutz, P. J., Lair, S. V. and Cregg, J. M. (1997) Isolation of the Pichia pastoris glyceraldehyde-3-phosphate dehydrogenase gene and regulation and use of its promoter. Gene 186, 37-44), le promoteur GAP en culture sur glucose semble plus fort que le promoteur AOX1 en culture sur méthanol, lors de cultures en Erlenmeyer.

Pour compléter cette phase de sélection, les clones PIC81 et PIC61 d'une part et GAP29 et GAP66 d'autre part ont été testés en fermenteur. Pour les clones PIC, deux batch successifs sur glycérol (40 g/L) permettent d'obtenir une biomasse élevée avant induction par le méthanol (780 g/L) en mode fed-batch. Pour les clones GAP, une première culture batch sur glucose (20 g/L) est suivie d'une phase de production en mode fed-batch sur glucose 400 g/L. Le clone PIC81 permet d'obtenir une biomasse finale de 81 g MS/L alors que la biomasse finale du clone PIC61 n'est que de 65 g MS/L. Par contre, les deux clones produisent sensiblement la même quantité de recPhyt (100 U/mL). Les clones GAP présentent des croissances identiques (100 g MS/L) en fin de culture mais les productions obtenues sont de 11 U/mL pour le clone GAP29 et de 9 U/mL pour le clone GAP66. Les souches recombinantes obtenues produisent respectivement 100 (souches PIC) et 10 (souches GAP) fois plus de phytase que la souche sauvage *D. castellii* CBS 2923. La production par les clones PIC81 et GAP29 a été optimisée en fermenteur.

### Production de phytase recombinante

### Expression constitutive - Influence de la source de carbone et de la vitesse spécifique de croissance sur la production du clone GAP

La production de recPhyt a été étudiée en culture sur glucose (400 g/L), glycérol (400 g/L) et méthanol (780 g/L) en mode fed-batch. Les résultats sont présentés dans le **tableau 8.**

**Tableau 8 : Comparaison des paramètres obtenus lors des cultures fed-batch du cloneGAP29. Pour toutes les expériences, les phases batch ont été réalisées sur le même milieu contenant 40 g/L de glycérol.**

| **Substrat pour le Fed-batch** | µ **imposé h⁻¹** | **Phase de culture** | **Biomas se g MS/L** | **Yx/ s g/g** | µ **global h⁻¹** | **recPh yt U/mL** | **YrecPhyt /x U/g MS** |
|---|---|---|---|---|---|---|---|
| Glucose | 0.010 | Batch (49 h)^{a} | 19.3 | 0.5 | 0.20 | 6.5 | 340 |
| | | Fed-batch (94 h)^{a} | 34.6. | 0.3 | 0.009 | 10 | 289 |
| | | *Final (143 h)*^{b} | *53.9* | | | *16.5* | *306* |
| Glycérol | 0.010 | Batch (49 h)^{a} | 20.5 | 0.5 | 0.20 | 7 | 341 |
| | | Fed-batch (94 h)^{a} | 35.2 | 0.3 | 0.008 | 9.6 | 272 |
| | | *Final (143 h)*^{b} | *55.7* | | | *16.6* | *298* |
| Glucose | 0.040 | Batch (26 h)^{a} | 19.2 | 0.5 | 0.20 | 5 | 260 |
| | | Fed-batch phase 1 (38 | 44.6 | 0.56 | 0.037 | 7.1 | 159 |
| | | Fed-batch phase 2 (24 h)^{a} | 17.7 | 0.38 | 0.016 | 3.9 | 220 |
| | | *Final (88 h)*^{b} | *81.5* | | | *16* | *196* |
| Glycérol | 0.040 | Batch (31 h)^{a} | 20.3 | 0.5 | 0.20 | 6 | 295 |
| | | Fed-batch phase 1 (38.5 h)^{a} | 45.3 | 0.55 | 0.039 | 7.3 | 161 |
| | | Fed-batch phase 1 (24 h)^{a} | 13.2 | 0.3 | 0.016 | 3.3 | 250 |
| | | *Final (93.5h)*^{b} | *78.8* | | | *16.6* | *210* |
| Méthanol | 0.010 | Batch (29 h)^{a} | 40 | 0.5 | 0.15 | 9.6 | 240 |
| | | Fed-batch (113 h)^{a} | 43.8 | 0.16 | 0.009 | 4.9 | 112 |
| | | *Final (142 h)*^{b} | *83.8* | | | *14.5* | *173* |

| | | | | | | | |
|---|---|---|---|---|---|---|---|
| ^{a}: Ces valeurs correspondent aux valeurs obtenues pendant chaque phase. ^{b}: Ces valeurs correspondent aux valeurs en fin de culture. | | | | | | | |

Pour les deux premiers substrats, un seul batch sur glycérol (40 g/L) permet d'atteindre 20 g/L de biomasse et une vitesse spécifique de croissance de 0.2 h⁻¹. Selon la durée de la phase stationnaire avant le fed-batch, la production de recPhyt est différente : voisine de 7 U/mL soit 340 U/g MS pour une phase stationnaire de 12 h, voisine de 5.5 U/mL soit 275 U/g MS pour une phase stationnaire de 3 h. La production en mode fed-batch sur glucose et glycérol avec une vitesse spécifique de croissance imposée de 0.01 h⁻¹ permet d'atteindre 55 g/L de biomasse, une production de phytase de 16.5 U/mL soit 280 U/g MS. Lors de la culture fed-batch sur méthanol après deux batch sur glycérol (40 g/L) et une vitesse spécifique de croissance imposée de 0.01 h⁻¹, la biomasse atteinte est de 84 g/L, la production de phytase de 14.5 U/mL soit 112 U/g MS. La production en mode fed-batch sur glucose et glycérol avec une vitesse spécifique de croissance imposée de 0.04 h⁻¹ permet d'atteindre 80 g/L de biomasse, une production de phytase de 16.5 U/mL soit 200 U/g MS.

Dans le cas où la production de phytase est sous le contrôle du promoteur GAP, le glucose et le glycérol sont de meilleurs substrats pour la production de phytase recombinante par rapport au méthanol, plus favorable à la croissance.

### Expression inductible - Influence de la vitesse spécifique de croissance sur la production du clone PIC

La production par le clone PIC81 a été étudiée en mode fed-batch (**figure 6** et **tableau 9**) et en mode continu (figure 6 et **tableau 9**).

**Tableau 9 : Comparaison des paramètres obtenus lors des cultures fed-batch et continu du clone PIC81**

| **Mode de culture** | | µ **ou D h⁻¹** | **Biomasse g MS/L** | **Yx/s g/g** | **recPhyt U/mL** | **YrecPhyt/x U/g MS** |
|---|---|---|---|---|---|---|
| Fed-batch 0.01 h¹ | phase 1 (64 h)^{a} | 0.012 | 28.8 | 0.21 | 34.7 | 1105 |
| | phase 2 (54 h)^{a} | 0.011 | 22.9 | 0.18 | 72.3 | 2342 |
| | *Final (118 h)*^{b} | | *51.7* | | *107* | *1337* |
| Fed-batch 0.03h⁻¹ | phase 1 (22.5 h)^{a} | 0.047 | 32.5 | 0.41 | 16.7 | 472 |
| | phase 2 (28.5 h)^{a} | 0.033 | 49.6 | 0.28 | 47.2 | 880 |
| | *Final (51 h)*^{b} | | *82.1* | | *63.9* | *614* |
| Continu^{c} | | 0.032 | 21.6 | 0.32 | 57.7 | 2700 |
| | | 0.053 | 27.2 | 0.41 | 11.6 | 430 |
| | | 0.077 | 26.3 | 0.39 | 5.7 | 210 |
| | | 0.095 | 26.6 | 0.40 | 3 | 116 |

| | | | | | | |
|---|---|---|---|---|---|---|
| ^{a}: Ces valeurs correspondent aux valeurs obtenues pendant chaque phase. ^{b}: Ces valeurs correspondent aux valeurs en fin de culture. ^{c}: Pour chaque taux de dilution, les mesures ont été réalisées après au moins trois renouvellements du contenu du fermenteur. | | | | | | |

En culture fed-batch, deux valeurs de vitesse spécifique de croissance (µ) ont été imposées (0.01 h⁻¹ et 0.03 h⁻¹). La biomasse obtenue est 1.5 fois plus élevée à µ = 0.03 h⁻¹ (82 g/L) qu'à µ = 0.01 h⁻¹ (52 g/L). La production de phytase s'effectue en deux phases (**tableau 9**). Pour une vitesse spécifique de croissance imposée de 0.01 h⁻¹, une première phase conduit à la production de 28.8 g/L de biomasse avec une production de phytase de 1105 U/g MS. Durant la deuxième phase, 22.9 g/L de biomasse sont obtenus et la production de phytase atteint 2342 U/g MS soit 107 U/mL. Des résultats similaires sont obtenus à µ = 0.03 h⁻¹, où la production de phytase passe de 472 U/g MS durant la première phase à 880 U/g MS pendant la deuxième phase soit 64 U/mL. L'augmentation de la vitesse spécifique de croissance est plus favorable à la croissance cellulaire qu'à la production de phytase recombinante. En culture continue, quatre taux de dilution ont été imposés (**tableau 9**). Pour des valeurs de taux de dilution supérieures à 0.053 h⁻¹, la biomasse à l'équilibre est de 27 g/L alors qu'elle n'est que de 21.6 g/L à un taux de dilution de 0.032 h⁻¹. Comme en culture en mode fed-batch, l'augmentation du taux de dilution est favorable à la production de biomasse au détriment de la production de protéines recombinantes. Les valeurs maximales, 2700 U/g MS soit 57.7 U/mL sont obtenues pour un taux de dilution de 0.032 h-¹. La comparaison de la production de phytase par un système inductible (promoteur AOX1, clone PIC81) et par un système constitutif (promoteur GAP, clone GAP29) montre que le promoteur AOX1 est nettement supérieur au promoteur GAP. La souche PIC81 a permis d'obtenir 107 U/mL de phytase recombinante soit 7 fois plus que la souche GAP29 (16 U/mL). La production spécifique est de respectivement 2700 U/g MS (PIC 81) et 340 U/g MS (GAP29) soit 100 et 10 fois supérieure à celle de la souche *D. castellii.*

### Purification

Le surnageant obtenu après une des cultures en mode fed-batch du clone PIC81 a été purifié en une étape comme décrit dans la partie Matériels et Méthodes. L'activité phytasique spécifique est de 101 U/mg de protéines dans l'extrait brut et de 182 U/mg de protéines dans l'extrait purifié. Ainsi, le facteur de purification est de 1.8. Cette valeur relativement faible suggère que la phytase recombinante est la protéine majoritaire dans l'extrait brut. Cette hypothèse a été confirmée en réalisant une électrophorèse SDS-PAGE. Sur le gel, la bande correspondant à la phytase recombinante représente 60 % des protéines totales. Par ailleurs, après purification, une seule bande est observée confirmant ainsi la purification à homogénéité de la protéine.

L'activité spécifique obtenue après purification est supérieure à celle obtenue lors de l'expression de la phytase d'*Aspergillus fumigatus* dans *P. pastoris* (43 U/mg de protéines) (Rodriguez, E., Mullaney, E. J. and Lei, X. G. (2000) Expression of the Aspergillus fumigatus phytase gene in Pichia pastoris and characterization of the recombinant enzyme. Biochem. Biophys. Res. Commun. 268, 373-378).

### Caractérisation de la phytase recombinante

### Effets du pH

RecPhyt est active pour des valeurs de pH comprises entre 2 et 6.5, et l'optimum se situe entre 4 et 4.5. La phytase de *D. castellii* CBS 2923 présente des caractéristiques similaires.

### Effets de la température

Les déterminations d'activité phytasique à différentes températures montrent que la phytase recombinante est plus active pour les températures élevées, avec un optimum à +60°C, comme la phytase de *D. castellii* CBS 2923. La plupart des valeurs citées pour la température optimum sont inférieures à +45°C, sauf pour quelques champignons et levures comme *Aspergillus ficuum* (+58°C) (Ullah, A. H. J. and Gibson, D. M. (1987) Extracellular phytase (EC 3.1.3.8) from Aspergillus ficuum NRRL 3135 : purification and characterization. Prep. Biochem. 17, 63-91), *Aspergillus terreus* (+70°C) (Yamada, K., Minoda, Y. and Yamamoto, S. (1968) Phytase from Aspergillus terreus. 1. Production, purification and some general properties of the enzyme. Agric. Biol. Chem. 32, 1275-1282) et *S. castellii* (+77°C) (Segueilha, L., Lambrechts, C., Boze, H., Moulin, G. and Galzy, P. (1992) Purification and properties of the phytase from Schwanniomyces castellii. J. Ferment. Bioeng. 74, 7-11). La représentation d'Arrhénius permet de calculer l'énergie d'activation de la réaction, E=37,9 kJ/mol. L'étude de la dénaturation thermique montre que la phytase recombinante est stable pour des températures inférieures à +67°C et fortement dénaturée pour des températures supérieures à +70°C. L'énergie d'activation calculée grâce à la représentation d'Arrhenius est de E=794,2 kJ/mol. La phytase de *D. castellii* CBS 2923 présente des caractéristiques de thermorésistance similaires.

### Spécificité de substrat

La phytase recombinante suit une cinétique de Michaelis-Menten, avec un Kₘ de 0.24 mM et une Vₘ de 137.8 U/mg sur le phytate de sodium et un Kₘ de 2.05 mM et une Vₘ de 274.6 U/mg sur le p-NPP (détermination grâce à la représentation de Lineweaver-Burk; données non publiées). Ainsi, l'enzyme a une plus grande affinité pour le phytate. La phytase de *D. castellii* CBS 2923 a des valeurs de Kₘ similaires.

### Etudes cinétiques

Les produit principaux de la dégradation de l'InsP₆ sont le DL-Ins(1,2,4,5,6)P₅ et le DL-Ins(1,2,5,6)P₄. La phytase recombinante est donc une 3-phytase. Par ailleurs, le chromatogramme montre que la dégradation des InsP₅, InsP₄, InsP₃ et InsP₂ commence avant même que l'InsP₆ soit épuisé et qu'il y a accumulation de I'InsP₃. Ceci suggère que la phytase recombinante a une moins forte affinité pour I'InsP₃ que pour l'InsP₄, I'InsP₅ et l'InsP₆. La phytase de *D. castellii* CBS 2923 montre les mêmes profils chromatographiques.

## Revendications

1. Polypeptide **caractérisé en ce qu'**il comprend un polypeptide choisi parmi les polypeptides suivants :
- le polypeptide de la SEQ ID No. 2 ,
- un fragment du polypeptide de la SEQ ID No. 2 ayant une activité phytase,
- un polypeptide ayant une activité phytase et présentant au moins 90 % d'identité avec le polypeptide de la SEQ ID No. 2.

2. Polynucléotide codant pour une activité phytase **caractérisé en ce qu'**il est choisi parmi les polynucléotides suivants :
- le polynucléotide dont la séquence est comprise entre la position 1538 et la position 2923 de la SEQ ID No. 1,
- un polynucléotide codant pour un polypeptide selon la revendication 1.

3. Polynucléotide **caractérisé en ce qu'**il a la séquence de la SEQ ID No. 1 ou la séquence complémentaire à la SEQ ID No. 1.

4. Cassette d'expression **caractérisée en ce qu'**elle comprend dans le sens de la transcription :
- un promoteur fonctionnel dans un organisme hôte;
- un polynucléotide selon la revendication 2; et
- une séquence terminatrice dans le même organisme hôte.

5. Vecteur comprenant un polynucléotide selon l'une des revendications 2-3 et/ou une cassette d'expression selon la revendication 4.

6. Procédé de transformation d'un organisme hôte par intégration dans ledit organisme hôte
- d'au moins un polynucléotide selon l'une des revendications 2 ou 3,
- d'une cassette d'expression selon la revendication 4, ou
- d'un vecteur selon la revendication 5
ledit organisme hôte étant *Pichia pastoris.*

7. Additif nutritionnel pour animaux **caractérisé en ce qu'**il comprend un polypeptide selon la revendication 1.

8. Additif nutritionnel pour animaux **caractérisé en ce qu'**il comprend un organisme hôte selon la revendications 6 et/ou un moût de fermentation d'un organisme hôte selon la revendications 6.

9. Additif nutritionnel pour animaux selon l'une des revendications 7 ou 8 **caractérisé en ce qu'**il se présente sous forme liquide ou sous forme de poudre.

10. Aliment pour animaux **caractérisé en ce qu'**il comprend une base nutritionnele pour animaux et un additif nutritionnel pour animaux selon l'une des revendications 7-9.

11. Aliment pour animaux **caractérisé en ce qu'**il comprend un polypeptide selon la revendication 1, un organisme hôte obtenu selon le procédé de la revendication 6 et/ou un moût de fermentation d'un organisme hôte obtenu selon le procédé de la revendication 6.

12. Utilisation d'un polypeptide selon la revendication 1 ou d'un organisme hôte obtenu selon le procédé de la revendication 6 pour la fabrication d'un additif nutritionnel pour animaux ou d'un aliment pour animaux.

13. Utilisation d'un polypeptide selon la revendication 1 ou d'un organisme hôte obtenu selon le procédé de la revendication 6 pour l'hydrolyse du myo-inositol hexakisphosphate en monophosphate inorganique, en myo-inositol de degré de phosphorylation inférieur et en myo-inositol libre.

## Claims

1. Polypeptide **characterized in that** it comprises a polypeptide chosen from the following polypeptides:
- the polypeptide of SEQ ID No. 2,
- a fragment of the polypeptide of SEQ ID No. 2 having phytase activity,
- a polypeptide having phytase activity and exhibiting at least 90% identity with the polypeptide of SEQ ID No. 2.

2. Polynucleotide encoding a phytase activity, **characterized in that** it is chosen from the following polynucleotides:
- the polynucleotide whose sequence is between position 1538 and position 2923 of SEQ ID No. 1,
- a polynucleotide encoding a polypeptide according to Claim 1.

3. Polynucleotide **characterized in that** it has the sequence of SEQ ID No. 1 or the sequence complementary to SEQ ID No. 1.

4. Expression cassette **characterized in that** it comprises, in the direction of transcription:
- a promoter that is functional in a host organism;
- a polynucleotide according to Claim 2; and
- a terminator sequence in the same host organism.

5. Vector comprising a polynucleotide according to either of Claims 2 and 3 and/or an expression cassette according to Claim 4.

6. Method for transformation of a host organism by integration, into said host organism:
- of at least one polynucleotide according to either of Claims 2 and 3;
- of an expression cassette according to Claim 4, or
- of a vector according to Claim 5, said host organism being *Pichia pastoris.*

7. Nutritional additive for animals, **characterized in that** it comprises a polypeptide according to Claim 1.

8. Nutritional additive for animals, **characterized in that** it comprises a host organism according to Claim 6 and/or a fermentation must of a host organism according to Claim 6.

9. Nutritional additive for animals according to either of Claims 7 and 8, **characterized in that** it is in liquid form or in powdered form.

10. Animal feed **characterized in that** it comprises a nutritional base for animals and a nutritional additive for animals according to one of Claims 7 to 9.

11. Animal feed **characterized in that** it comprises a polypeptide according to Claim 1, a host organism obtained according to the method of Claim 6 and/or a fermentation must of a host organism obtained according to the method of Claim 6.

12. Use of a polypeptide according to Claim 1 or of a host organism obtained according to the method of Claim 6, for the manufacture of a nutritional additive for animals or of an animal feed.

13. Use of a polypeptide according to Claim 1 or of a host organism obtained according to the method of Claim 6, for the hydrolysis of myo-inositol hexakisphosphate to inorganic monophosphate, to myoinositol with a lower degree of phosphorylation and to free myo-inositol.

## Patentansprüche

1. Polypeptid, **dadurch gekennzeichnet, dass** es ein Polypeptid enthält, das aus den folgenden Polypeptiden ausgewählt ist:
- dem Polypeptid von SEQ ID Nr. 2,
- einem Fragment des Polypeptids von SEQ ID Nr. 2 mit einer Phytaseaktivität,
- einem Polypeptid mit einer Phytaseaktivität, das mindestens 90% Identität mit dem Polypeptid von SEQ ID Nr. 2 aufweist.

2. Polynukleotid, das für eine Phytaseaktivität codiert, **dadurch gekennzeichnet, dass** es aus den folgenden Polynukleotiden ausgewählt ist:
- dem Polynukleotid, dessen Sequenz zwischen Position 1538 und Position 2923 von SEQ ID Nr. 1 umfasst ist,
- einem Polynukleotid, das für ein Polypeptid nach Anspruch 1 codiert.

3. Polynukleotid, **dadurch gekennzeichnet, dass** es die Sequenz von SEQ ID Nr. 1 oder die zu SEQ ID Nr. 1 komplementäre Sequenz aufweist.

4. Expressionskassette, **dadurch gekennzeichnet, dass** sie in Transkriptionsrichtung Folgendes umfasst:
- einen in einem Wirtsorganismus funktionsfähigen Promoter;
- ein Polynukleotid nach Anspruch 2; und
- eine Terminatorsequenz in demselben Wirtsorganismus.

5. Vektor, umfassend ein Polynukleotid nach einem der Ansprüche 2-3 und/oder eine Expressionskassette nach Anspruch 4.

6. Verfahren zum Transformieren eines Wirtsorganismus durch Integrieren
- von mindestens einem Polynukleotid nach einem der Ansprüche 2 oder 3,
- von einer Expressionskassette nach Anspruch 4, oder
- von einem Vektor nach Anspruch 5
in diesen Organismus, wobei es sich bei dem Wirtsorganismus um *Pichia pastoris* handelt.

7. Ernährungszusatz für Tiere, **dadurch gekennzeichnet, dass** er ein Polypeptid nach Anspruch 1 umfasst.

8. Ernährungszusatz für Tiere, **dadurch gekennzeichnet, dass** er einen Wirtsorganismus nach Anspruch 6 und/oder eine Fermentationsbrühe eines Wirtsorganismus nach Anspruch 6 umfasst.

9. Ernährungszusatz für Tiere nach einem der Ansprüche 7 oder 8, **dadurch gekennzeichnet, dass** er in flüssiger Form oder in Form eines Pulvers vorliegt.

10. Tiernahrung, **dadurch gekennzeichnet, dass** sie eine Ernährungsgrundlage für Tiere und einen Ernährungszusatz für Tiere nach einem der Ansprüche 7-9 umfasst.

11. Tiernahrung, **dadurch gekennzeichnet, dass** sie ein Polypeptid nach Anspruch 1, einen Wirtsorganismus, der nach dem Verfahren von Anspruch 6 erhalten worden ist, und/oder eine Fermentationsbrühe eines Wirtsorganismus, der nach dem Verfahren von Anspruch 6 erhalten worden ist, umfasst.

12. Verwendung eines Polypeptids nach Anspruch 1 oder eines Wirtsorganismus, der nach dem Verfahren von Anspruch 6 erhalten worden ist, für die Herstellung eines Ernährungszusatzes für Tiere oder einer Nahrung für Tiere.

13. Verwendung eines Polypeptids nach Anspruch 1 oder eines Wirtsorganismus, der nach dem Verfahren von Anspruch 6 erhalten worden ist, für die Hydrolyse von Myo-Inositolhexakisphosphat zu anorganischem Monophosphat, zu Myo-Inositol mit einem niedrigeren Phosphorylierungsgrad und zu freiem Myo-Inositol.
